# EUROPEAN PATENT SPECIFICATION

(11) **EP 2 591 715 B1**
(45) Date of publication and mention of the grant of the patent: **02.08.2017**
(21) Application number: 11803471.9
(22) Date of filing: 28.06.2011
(51) Int. Cl.: A61B 1/00, A61B 1/04, A61B 1/045

(54) **ENDOSCOPE SYSTEM AND CONTROL METHOD OF THE ENDOSCOPE SYSTEM**
ENDOSKOPSYSTEM UND STEUERUNGSVERFAHREN DES ENDOSKOPSYSTEMS
SYSTÈME D'ENDOSCOPE ET PROCÉDÉ DE COMMANDE DU SYSTÈME D'ENDOSCOPE

(30) Priority: 07.07.2010 JP 2010155236
(43) Date of publication of application: 15.05.2013
(73) Proprietor: Olympus Corporation, Tokyo 192-8507 (JP)
(72) Inventor: GOCHO, Masanori, Hachioji-shi, Tokyo 192-8507 (JP); KONDOH, Masaki, Hachioji-shi, Tokyo 192-8507 (JP)
(74) Representative: Gunzelmann, Rainer
(86) International application number: PCT/JP2011/064731
(87) International publication number: WO 2012/005136

(56) References cited:
- EP-A1- 1 685 788
- JP-A- 2 135 406
- JP-A- 4 204 638
- JP-A- 2003 325 439
- JP-A- 2003 325 439
- JP-A- 2005 334 116
- JP-A- 2006 055 538
- JP-A- 2006 157 505
- JP-A- 2008 154 934
- JP-A- 2008 526 289
- JP-A- 2008 529 631
- US-A1- 2010 141 744
- US-B1- 6 436 032
- US-B1- 6 638 212

## Description

### Background of the Invention

### Field of the Invention

The present invention relates to an endoscope system, and a control method of the endoscope system.

### Technical Background of the Invention

Endoscope systems are widely used, for example, in a medical field, an industrial field, and the like. Such endoscope systems are normally composed of a scope of diverse functions, which includes an image capturing unit for shooting an image to be examined, and an image processing device for visualizing and displaying the shot image by controlling the scope.

To enable a control suitable for an image processing device tailored for specifications of each scope, a nonvolatile memory is provided on the side of each scope, specific parameters of each scope are stored in the nonvolatile memory, and the image processing device reads and uses these parameters, so that controls suitable for diverse scopes are attempted to be implemented as referred to in Patent Document 1.

Incidentally, the amount of data of specific parameters stored in a nonvolatile memory on the side of a scope has been increasing with recent performance improvements, function diversification, and the like of endoscope systems.

With the conventional technique disclosed by Patent Document 1, data is read from the side of a scope to the side of an image processing device with a serial communication made between processors respectively arranged on the side of the scope and the side of the image processing device. However, in a serial communication made between CPUs, a considerable amount of time is needed to transfer data, and a signal different from a video signal is used. This can possibly cause a noise of a displayed image.

Accordingly, Patent Document 2 discloses a technique of attempting to solve the technical problem of a noise by making a communication between a scope and an image processing device as a start-stop synchronous communication that is synchronized with an operation clock of an image capturing element.

### [Prior Art Documents] [Patent Documents]

[Patent Document 1] Japanese Laid-open Patent Publication No. 2000-284957
[Patent Document 2] Japanese Laid-open Patent Publication No. 2010-81975

US 2010/0141744 A1 discloses a system for wirelessly powering various devices positioned on an endoscope, including, a light source, various electronics including an imager and/or a memory device. The system is further provided such that video signal processing parameters are automatically set for an endoscopic video camera system based upon characteristics of an attached endoscope, with reduced EMI and improved inventory tracking, maintenance and quality assurance, and reducing the necessity for adjustment and alignment of the endoscope and camera to achieve the data transfer.

US 6,638,212 B1 discloses a nonvolatile, programmable memory incorporated in an endoscope. Endoscope-related data closely relevant to the endoscope, such as, an endoscope model name and the number of power feeds are stored in the nonvolatile memory. The endoscope is connected to an external image processing apparatus, and endoscope-related data is read from the nonvolatile memory. Based on the read endoscope-related data, the use situation of the endoscope is grasped or the endoscope is managed.

### Summary of the Invention

### Problems to be Solved by the Invention

However, the technique disclosed by Patent document 2 has a technical problem such that a considerable amount of time is needed to transfer a large amount of data stored in a nonvolatile memory on the side of the scope since the communication is made in a blanking interval.

Moreover, as the endoscope system, it is needed to communicate a value of an electronic shutter, switch information included in the scope, and the like between the scope and the image processing device in addition to the above described specific parameters as occasion demands. Because of this , it is insufficient to establish an endoscope system ,with merely transferring the data of the specific parameters stored in the nonvolatile memory.

There is also a technical problem of reducing the number of pins of signal lines in an interface connecting the scope and the image processing device.

An object of the present invention is to provide a technique by which diverse items of data respectively having different attributes and data amounts can be efficiently transferred in a short time without increasing the number of transmission paths between a scope unit and an image processing unit, which configure an endoscope system.

### Means for Solving the Problems

A first aspect of the present invention provides an endoscope system having the features of claim 1.

A second aspect of the present invention provides a control method of an endoscope system having the features of claim 4.

### Advantages of the Invention

According to the present invention, diverse pieces of data respectively having different attributes and data amounts can be efficiently transmitted in a short time without increasing the number of transmission paths between the scope unit and the image processing unit, which configure the endoscope system.

### Brief Description of the Drawings

FIG. 1 is a block diagram illustrating one example of a configuration of an endoscope system for implementing a control method of the endoscope system according to an embodiment of the present invention;
FIG. 2 is a conceptual schematic illustrating one example of configurations of data formats of transmission/reception packets used in the control method of the endoscope system according to the embodiment of the present invention;
FIG. 3 is a conceptual schematic illustrating an example of a configuration of a sequence flag portion in the transmission/reception packet used in the control method of the endoscope system according to the embodiment of the present invention;
FIG. 4 is a state transition diagram illustrating one example of actions of a hardware circuit for performing a transmission operation on the side of an image processing device in the control method of the endoscope system according to the embodiment of the present invention;
FIG. 5 is a state transition diagram illustrating one example of actions of a hardware circuit for performing a reception operation on the side of the image processing device in the control method of the endoscope system according to the
   embodiment of the present invention;
FIG. 6 is a state transition diagram illustrating one example of actions of a hardware circuit for performing a reception operation on the side of a scope in the control method of the endoscope system according to the embodiment of the present invention;
FIG. 7 is a state transition diagram illustrating one example of actions of a hardware circuit for performing a transmission operation on the side of the scope in the control method of the endoscope system according to the embodiment of the present invention;
FIG. 8 is a conceptual schematic illustrating an example of a configuration of an endoscope system for implementing a control method of the endoscope system according to another embodiment of the present invention;
FIG. 9 is a conceptual schematic illustrating a modification example of the configuration of the endoscope system for implementing the control method of the endoscope system according to the another embodiment of the present invention;
FIG. 10 is a conceptual schematic illustrating a modification example of the configuration of the endoscope system for implementing the control method of the endoscope system according to the another embodiment of the present invention;
FIG. 11 is a conceptual schematic illustrating examples of structures of packets used in the endoscope system for implementing the control method of the endoscope system according to the another embodiment of the present invention;
FIG. 12 is a timing chart illustrating one example of actions of the endoscope system for implementing the control method of the endoscope system according to the another embodiment of the present invention;
FIG. 13 is a state transition diagram illustrating one example of actions of a hardware circuit for performing a transmission operation on the side of an image processing device in the control method of the endoscope system according to the another embodiment of the present invention;
FIG. 14 is a state transition diagram illustrating one example of actions of a hardware circuit for performing a reception operation on the side of the image processing device in the control method of the endoscope system according to the another embodiment of the present invention;
FIG. 15 is a state transition diagram illustrating one example of actions of a hardware circuit for performing a reception operation on the side of a scope in the control method of the endoscope system according to the another embodiment of the present invention;
FIG. 16 is a state transition diagram illustrating one example of actions of a hardware circuit for performing a transmission operation on the side of the scope in the control method of the endoscope system according to the another embodiment of the present invention;
FIG. 17 is a block diagram illustrating one example of a configuration of an endoscope system for implementing a control method of the endoscope system according to a further embodiment of the present invention; and
FIG. 18 is a block diagram illustrating one example of a configuration of an endoscope system for implementing a control method of the endoscope system according to a still further embodiment of the present invention.

### Description of the Preferred Embodiments

Embodiments according to the present invention are described in detail below with reference to the drawings.

### (Embodiment 1)

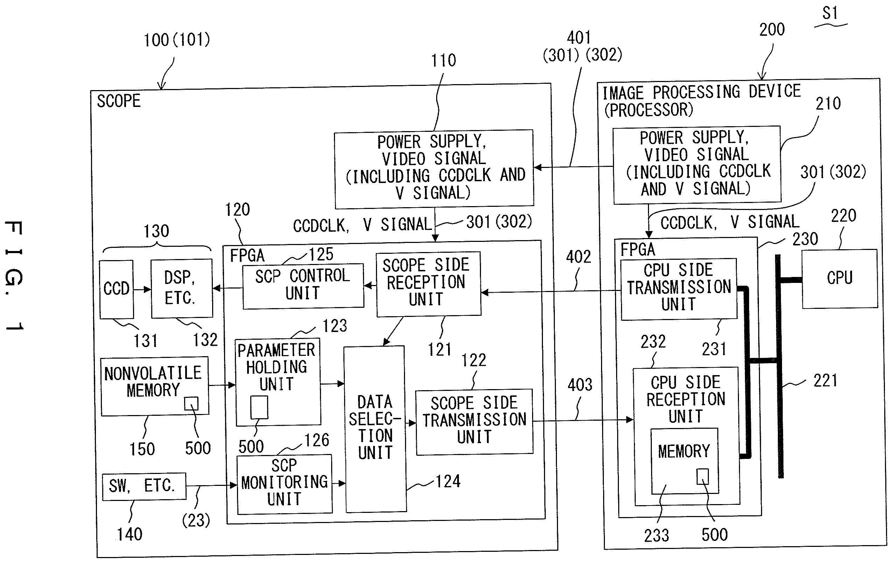

FIG. 1 is a block diagram illustrating one example of a configuration of an endoscope system for implementing a control method of the endoscope system according to an embodiment of the present invention.

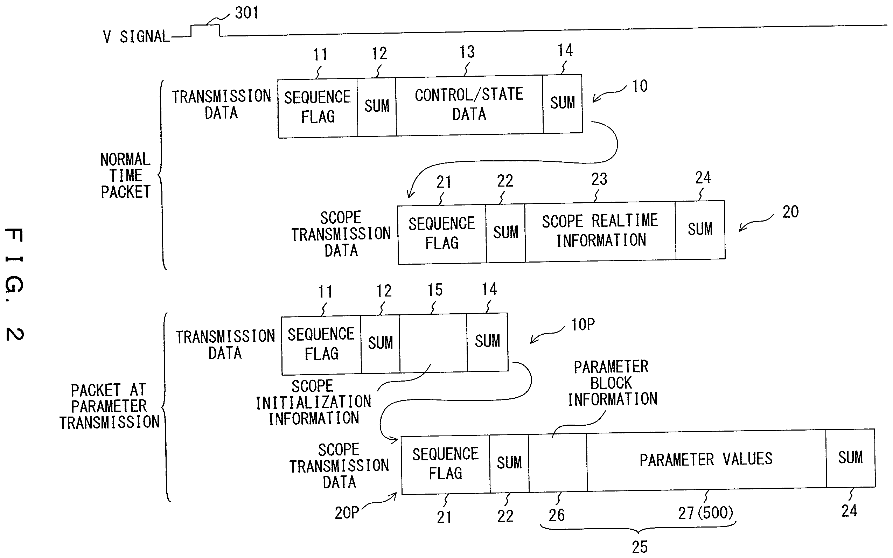

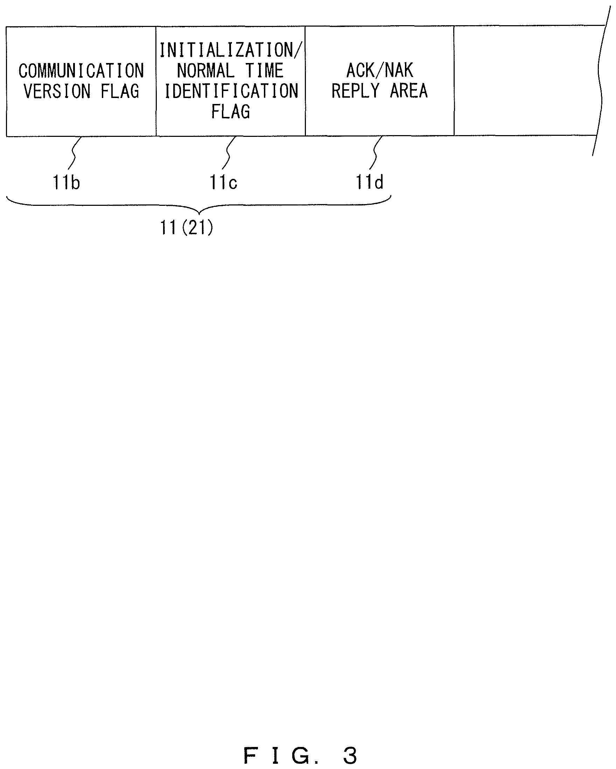

FIG. 2 is a conceptual schematic illustrating one example of configurations of data formats of transmission/reception packets used in the control method of the endoscope system according to the embodiment of the present invention, and FIG. 3 is a conceptual schematic illustrating an example of a configuration of sequence flag portions of the transmission/reception packets used in the control method of the endoscope system according to the embodiment of the present invention.

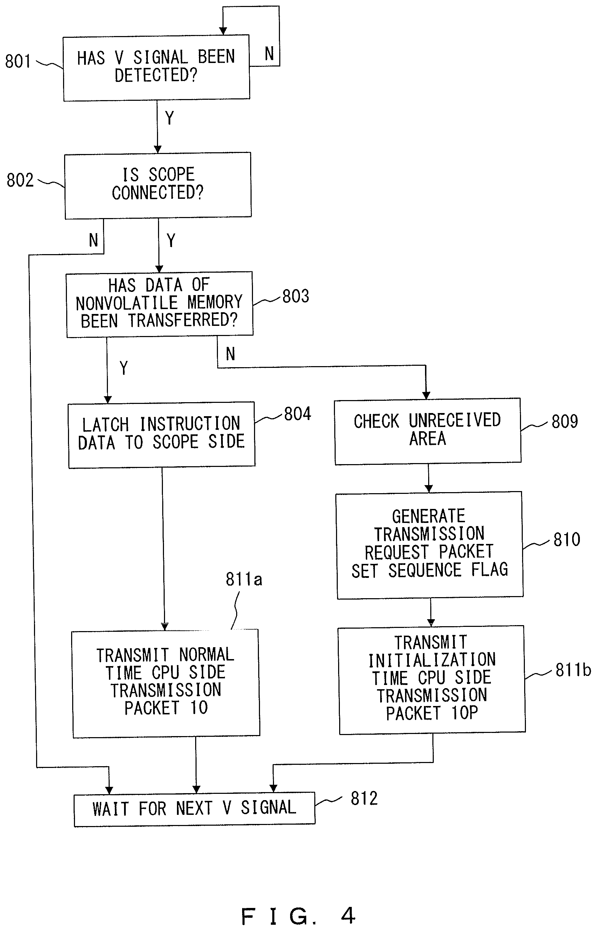

FIG. 4 is a state transition diagram illustrating one example of actions of a hardware circuit for performing a transmission operation on the side of an image processing device in the control method of the endoscope system according to the embodiment of the present invention.

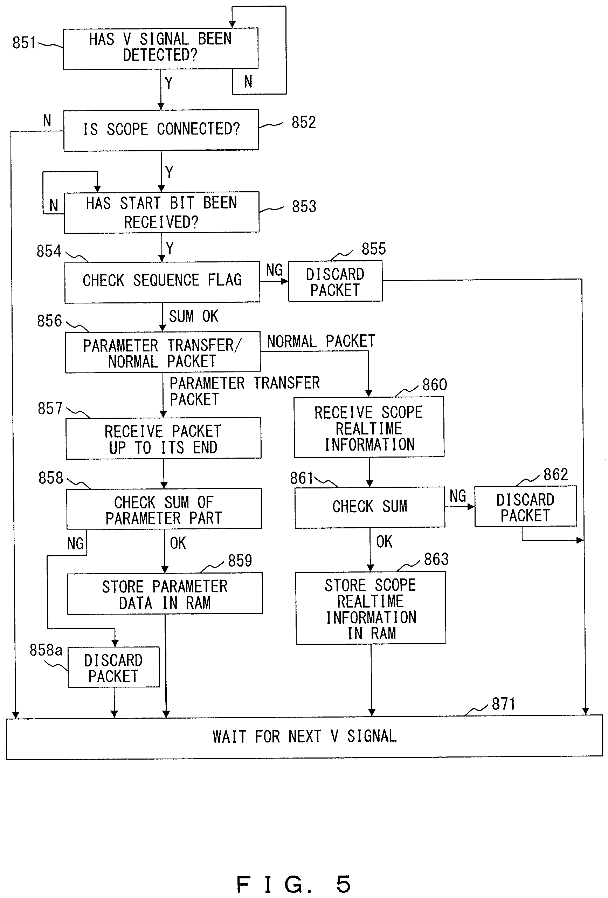

FIG. 5 is a state transition diagram illustrating one example of actions of a hardware circuit for performing a reception operation on the side of the image processing device in the control method of the endoscope system according to the embodiment of the present invention.

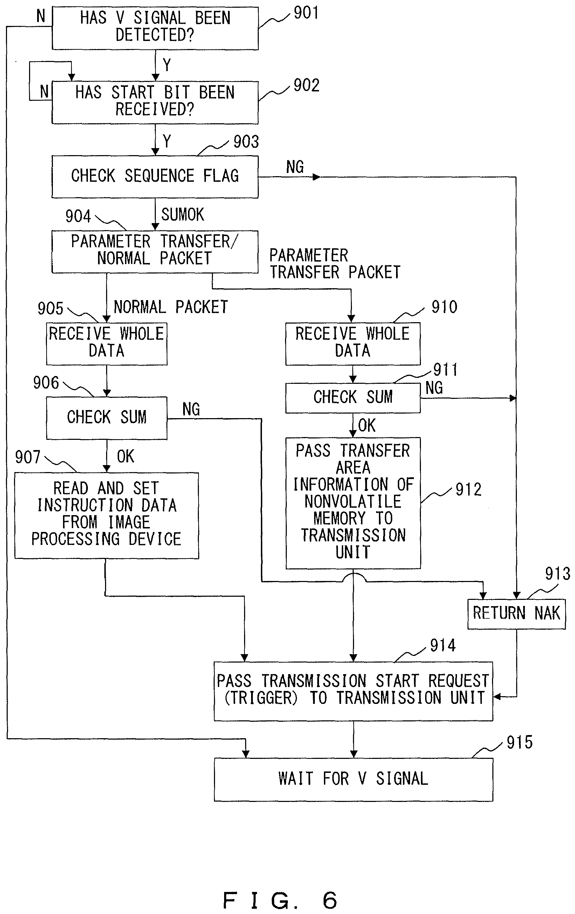

FIG. 6 is a state transition diagram illustrating one example of actions of a hardware circuit for performing a reception operation on the side of a scope in the control method of the endoscope system according to the embodiment of the present invention.

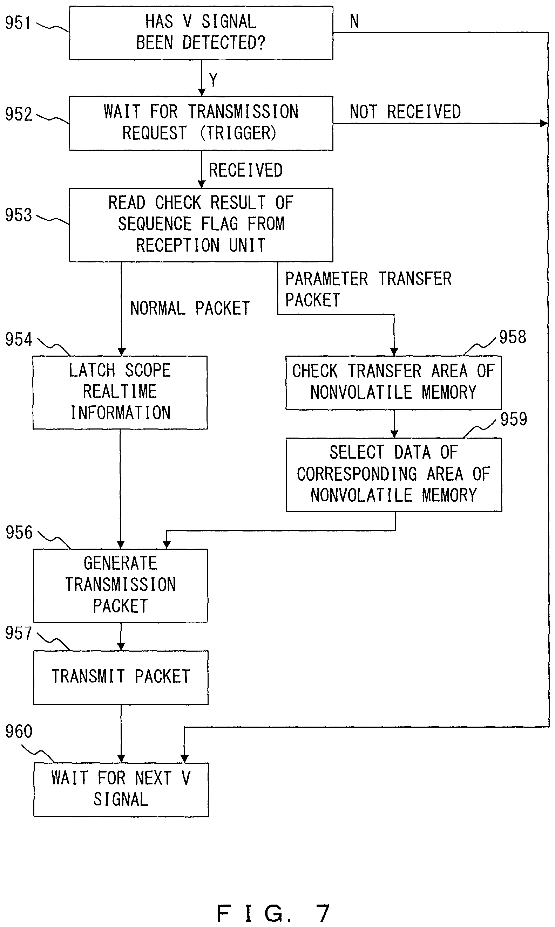

FIG. 7 is a state transition diagram illustrating one example of actions of a hardware circuit for performing a transmission operation on the side of the scope in the control method of the endoscope system according to the embodiment of the present invention.

As illustrated in FIG. 1, the endoscope system S1 according to this embodiment includes a scope 100 (scope unit) that configures an endoscope 101, and an image processing device 200 (image processing unit), connected to the scope 100, for controlling the scope 100.

The image processing device 200 includes a CPU 220 that controls the whole of the image processing device 200, a processor side control logic circuit 230 connected to the CPU 220 via a bus 221, and a power/clock supply circuit 210 for supplying a synchronization signal, a clock, and power to units of the image processing device 200.

In the case of this embodiment, the power/clock supply circuit 210 supplies a vertical synchronizing signal 301 (V signal), and an image capturing element driving clock 302 (CCDCLK) to the processor side control logic circuit 230.

The processor side control logic circuit 230 is configured, for example, with hardware such as an FPGA or the like. In the case of this embodiment, logic blocks such as a CPU side transmission unit 231 (first data transmission means), a CPU side reception unit 232 (first data reception means), and a parameter memory 233 are constructed.

In the meantime, the scope 100 includes a power/clock supply circuit 110, a scope side control logic circuit 120, an image capturing unit 130 (image capturing means), a scope operation unit 140, a nonvolatile memory 150, and the like.

The power/clock supply circuit 110 supplies power, the vertical synchronizing signal 301, and the image capturing element driving clock 302, which are received from the power/clock supply circuit 210 of the image processing device 200 via an interface line 401, to the units of the scope 100.

The image capturing unit 130 is configured with an image capturing element 131 and an image capturing element driving unit 132, and outputs a video signal of a target.

The video signal output from the image capturing unit 130 is conveyed to the image processing device 200 via a dedicated transmission interface, and displayed on a display that is connected to the image processing device 200 and not illustrated, although this is not particularly illustrated in FIG. 1.

The image capturing element driving unit 132 is configured with a DSP (digital signal processor) or the like for implementing functions such as an A/D conversion function of converting an analog video signal of the image capturing element 131 into a digital signal, a noise removal function, and a function of outputting and processing a digital video signal in conformity with a video standard such as NTSC, PAL or the like.

The scope operation unit 140 is configured with a switch with which a user operates the units of the scope 100 in the endoscope 101.

The nonvolatile memory 150 stores information such as specific parameters 500 of the scope 100, and the like persistently.

The scope side control logic circuit 120 is configured, for example, with hardware such as an FPGA or the like, and provides control logic for controlling the whole of the scope 100 based on the specific parameters 500 stored in the nonvolatile memory 150.

Specifically, in the case of this embodiment, the scope side control logic circuit 120 implements the logic blocks such as a scope side reception unit 121 (second data reception means), a scope side transmission unit 122 (second data transmission means), a parameter holding unit 123, a data selection unit 124, a scope control unit 125, and a scope monitoring unit 126 by using hardware.

The scope side reception unit 121 is connected to the CPU side transmission unit 231 of the image processing device 200 via a serial communication line 402, and has a function of receiving a packet such as a normal time CPU side transmission packet 10 (a first packet), an initialization time CPU side transmission packet 10P (first packet) or the like, which are transmitted from the CPU side transmission unit 231 and will be described later, and of distributing information extracted from the packet to the units.

The scope side transmission unit 122 is connected to the CPU side reception unit 232 of the image processing device 200 via a serial communication line 403, and has a function of conveying, to the side of the image processing device 200, the specific parameters 500 within the scope 100, and information occurred in the scope operation unit 140 in a format of a normal time scope side transmission packet 20 (in a second data format) (a second packet) or an initialization time scope side transmission packet 20P (in a first data format) (a second packet), which will be described later.

In the case of this embodiment, packet transmissions/receptions between the CPU side transmission unit 231 and the scope side reception unit 121, and between the scope side transmission unit 122 and the CPU side reception unit 232 are performed with a serial communication of a start-stop synchronous serial communication that is synchronized with the image capturing element driving clock 302 at timing of the vertical synchronizing signal 301 for controlling the image capturing element 131.

The parameter holding unit 123 is a buffer memory, configured, for example, with a memory block such as an SRAM, a DRAM or the like, for holding the specific parameters 500, to which a fast access can be made, by reading the parameters from the nonvolatile memory 150 configured with an EEPROM or the like.

The scope monitoring unit 126 is a logic block having a function of monitoring an operation and a state of the scope operation unit 140 such as a switch or the like, and of conveying the detected operation or state of the scope operation unit 140 to the data selection unit 124 as part of scope realtime information 23 (realtime information) to be described later.

The data selection unit 124 is a logic block having a function of structuring a packet by reading the specific parameters 500 of the parameter holding unit 123 and the scope realtime information 23 of the scope monitoring unit 126 based on a type of a packet at timing when the packet is received from the side of the CPU side transmission unit 231 via the scope side reception unit 121, and of transmitting the packet from the scope side transmission unit 122 to the CPU side reception unit 232.

The scope control unit 125 is a logic block having a function of setting information for controlling the operations of the image capturing unit 130, and of controlling an operation state based on information of control/state data 13 that is included in a packet received from the CPU side transmission unit 231 via the scope side reception unit 121 and will be described later.

Examples of structures of packets used in a communication between the scope 100 and the image processing device 200 in the endoscope system S1 according to this embodiment are described next with reference to FIGs. 2 and 3.

The endoscope system S1 according to this embodiment utilizes a combination of the initialization time CPU side transmission packet 10P and the initialization time scope side transmission packet 20P, which are used to transmit the specific parameters 500 from the scope 100 to the image processing device 200 at activation of the system, in addition to a combination of the normal time CPU side transmission packet 10 and the normal time scope side transmission packet 20, which are transmitted/received between the image processing device 200 and the scope 100 in a normal running state.

In the case of this embodiment, transmissions/receptions of these packets are performed by using the vertical synchronizing signal 301 as a trigger as will be described later.

As illustrated on an upper side of FIG. 2, the normal time CPU side transmission packet 10 that is transmitted from the image processing device 200 to the scope 100 at normal times has a configuration where a sequence flag 11, a checksum 12, control/state data 13, and a checksum 14 are arranged sequentially from the start side of the packet.

Additionally, the normal time scope side transmission packet 20 that is returned from the scope 100 to the image processing device 200 in response to the reception of the normal time CPU side transmission packet 10 has a configuration where a sequence flag 21, a checksum 22, scope realtime information 23, and a checksum 24 are arranged sequentially from the start side of the packet.

The sequence flag 11 and the sequence flag 21 on the start side have a common configuration, and is composed of a communication version flag 11b, an initialization/normal time identification flag 11c (identification information), and an ACK/NAK reply flag 11d as illustrated in FIG. 3.

A bit pattern, intended to make the scope 100 identify whether the packet is either a packet used in a communication at normal times or a transfer request of the specific parameters 500 at initialization, is set by the image processing device 200 in the initialization/normal time identification flag 11c of the sequence flag 11. Specifically, the flag is set to "1" if the packet is that used in a communication at normal times, or set to "0" if the packet is that used at initialization.

The ACK/NAK reply flag 11d of the sequence flag 21 is set on the side of the scope 100 so that whether the normal time CPU side transmission packet 10 is received either successfully or unsuccessfully is notified to the image processing device 200. A bit pattern indicating ACK is set if the packet is successfully received, or a bit pattern indicating NAK is set if the packet is unsuccessfully received.

The communication version flag 11b is common to the sequence flag 11 and the sequence flag 21, and information reserved for a future system change is set.

The checksum 12 of the normal time CPU side transmission packet 10 is used to check an error of the sequence flag 11.

The control/state data 13 is used by the image processing device 200 to notify the scope 100 of an operation state or to convey control information such as shutter data or the like at normal times. Specifically, the control/state data 13 includes information with which the side of the image processing device 200 changes a state on the side of the scope 100, such as a forcible rest of the DSP of the image capturing element driving unit 132, an electronic shutter, a gain, and the like.

The checksum 14 is used to check an error of the control/state data 13.

The checksum 22 of the normal time scope side transmission packet 20 is used to check an error of the sequence flag 21.

The scope realtime information 23 stores information indicating an operation state on the side of the scope 100 at normal times. Specifically, the scope realtime information 23 is information for transmitting, for example, a state of the switch operated in the scope operation unit 140 in the scope 100, a state of the DSP that configures the image capturing element driving unit 132 in the image capturing unit 130 to the side of the image processing device 200 in real time.

The checksum 24 is used to check an error of the scope realtime information 23.

In the meantime, the initialization time CPU side transmission packet 10P used to transfer the specific parameters 500 at initialization is the same as the normal time CPU side transmission packet 10 except that the initialization/normal time identification flag 11c of the sequence flag 11 is set to "0" that indicates initialization and the scope initialization information 15 is set as a replacement for the control/state data 13.

In the case of this embodiment, for example, information specifying which of NTSC and PAL is to be used to operate the image capturing unit 130 is set as the scope initialization information 15.

The initialization time scope side transmission packet 20P is configured by setting parameter reply information 25 as a replacement for the scope realtime information 23 in the above described normal time scope side transmission packet 20.

In the case of this embodiment, the parameter reply information 25 is composed of parameter block information 26 indicating a storage position, a transfer serial number and the like of parameter data in each transfer, and parameter values 27, which are values of specific parameters, when the specific parameters 500 in the nonvolatile memory 150 are divided into a plurality of packets and transferred.

An example of actions of the endoscope system S1 according to this embodiment is described below.

Initially, in the scope side control logic circuit 120 of the scope 100 according to this embodiment, at activation by power-on, a reset or the like, all the specific parameters 500 are copied from the nonvolatile memory 150 to the parameter holding unit 123 that is quickly accessible, and the scope side control logic circuit 120 operates by using the specific parameters 500 stored in the parameter holding unit 123.

Additionally, the scope side control logic circuit 120 makes a reply also to a transfer request issued from the image processing device 200 by using the specific parameters 500 stored in the parameter holding unit 123.

Namely, in the case of the endoscope system S1 according to this embodiment, the whole (or part) of data of the specific parameters 500 in the nonvolatile memory 150 is transferred to the side of the image processing device 200 as an initial operation by using the initialization time CPU side transmission packet 10P and the initialization time scope side transmission packet 20P after the scope 100 has been activated. The image processing device 200 holds the transferred data of the specific parameters 500 in the parameter memory 233, and references the data of the specific parameters 500 in the parameter memory 233 when needed.

Upon completion of the data transfer and the holding of the specific parameters 500 from the scope 100 to the image processing device 200, normal data such as the control/state data 13, the scope realtime information 23 and the like are transmitted/received between the scope 100 and the image processing device 200 by using the normal time CPU side transmission packet 10 and the normal time scope side transmission packet 20.

One example of transmission/reception operations of the above described packets of the scope 100 and the image processing device 200 in the endoscope system S1 according to this embodiment is described next with reference to FIGs. 4, 5, 6, and 7.

The transmission operation performed by the image processing device 200 is initially described with reference to FIG. 4.

The processor side control logic circuit 230 (the CPU side transmission unit 231) of the image processing device 200 determines whether or not the scope 100 is connected (step 802) at timing when a pulse of the vertical synchronizing signal 301 has been detected (step 801). If the processor side control logic circuit 230 determines that the scope 100 is connected, it further determines whether or not all the specific parameters 500 have been transferred from the scope 100 (step 803). This determination is performed, for example, based on information such as a serial number or the like of the parameter block information 26 of the parameter reply information 25 that is obtained from the received initialization time scope side transmission packet 20P and stored in the register or the like within the processor side control logic circuit 230.

If the processor side control logic circuit 230 determines that all the specific parameters 400 have been transferred, it configures control/state data 13 of the normal time CPU side transmission packet 10 by latching instruction data to the side of the scope 100 at normal times, and transmits the normal time CPU side transmission packet 10 from the CPU side transmission unit 231 to the scope side reception unit 121 of the scope 100 (step 811a).

Thereafter, the processor side control logic circuit 230 waits until the next vertical synchronizing signal 301 is detected (step 812).

In the meantime, if the processor side control logic circuit 230 determines that all the parameters 500 have not been transferred yet in the above described step 803, it checks an unreceived area of the specific parameters 500 based on the already received parameter block information 26 (step 809), generates an initialization time CPU side transmission packet 10P where the initialization/normal time identification flag 11c of the sequence flag 11 is set to "0" (step 801), and transmits the packet to the scope 100 (step 811b).

In contrast, if the processor side control logic circuit 230 determines in the above described step 802 that the scope 100 is not connected, it waits until the next vertical synchronizing signal 301 is detected in the above described step 812.

Additionally, as illustrated in FIG. 5, the CPU side reception unit 232 (the processor side control logic circuit 230) of the image processing device 200 determines whether or not the scope 100 is connected (step 852) at timing when the vertical synchronizing signal 301 is detected (step 851). If the CPU side reception unit 232 determines that the scope 100 is connected, it waits until a start bit of a packet (a normal time scope side transmission packet 20 or an initialization time scope side transmission packet 20P) is received from the scope 100 (step 853). Upon receipt of the start bit, the CPU side reception unit 232 checks a sequence flag 21 when it has received the packet from the sequence flag 21 up to the checksum 22 on the start side of the packet (step 854).

If the CPU side reception unit 232 determines that the packet has been unsuccessfully received based on the checksum 22, or if the ACK/NAK reply flag 11d of the sequence flag 21 is NAK although the packet has been successfully received, it discards the packet (step 855) and waits for the next vertical synchronizing signal (step 871).

In contrast, if the CPU side reception unit 232 determines that the packet has been successfully received based on the checksum of the sequence flag 21, it determines whether the packet is either the normal time scope side transmission packet 20 at normal times or the initialization time scope side transmission packet 20P for a parameter transfer by checking the initialization/normal time identification flag 11c of the sequence flag 21 (step 856).

If the packet is the initialization time scope side transmission packet 20P where the initialization/normal time identification flag 11c is set to "0", the CPU side reception unit 232 receives the initialization time scope side transmission packet 20P up to its end (step 857), and checks the checksum 24 of the parameter reply information 25 (step 858). If the packet has been successfully received, the CPU side reception unit 232 stores parameter values 27 of the parameter reply information 25 in the parameter memory 233, also stores, in a register or the like, information such as a serial number or the like of the parameter block information 26, which indicates a proceeding state of a divided transfer (step 859), and waits for the next vertical synchronizing signal 301 (step 871) .

If the CPU side reception unit 232 determines in the above described step 858 that the packet has been received unsuccessfully, it discards the received initialization time scope side transmission packet 20P (step 858a)

In contrast, if the CPU side reception unit 232 determines that the packet is a normal time scope side transmission packet 20 at normal times where the initialization/normal time identification flag 11c is set to "001" in the above described step 856, it reads the scope realtime information 23 and the checksum 24 or the like as the end of the normal time scope side transmission packet 20 (step 860), and checks the checksum 24 (step 861).

If the CPU side reception unit 232 determines that the packet has been successfully received, it stores scope realtime information 23 of the normal time scope side transmission packet 20 in part of the parameter memory 233 (step 863), and waits for the next vertical synchronizing signal 301 (step 871).

In contrast, if the CPU side reception unit 232 determines in step 861 that the packet has been unsuccessfully received, it discards the current normal time scope side transmission packet 20 (step 862), and waits for the next vertical synchronizing signal (step 871).

Examples of operations of the scope side reception unit 121 and the scope side transmission unit 122 in the scope side control logic circuit 120 of the scope 100 according to this embodiment are described next with reference to FIGs. 6 and 7.

As described above, in the scope side control logic circuit 120 of the scope 100 according to this embodiment, the specific parameters 500 within the nonvolatile memory 150 are pre-read into the parameter holding unit 123 at activation.

As illustrated in FIG. 6, the scope side control logic circuit 120 (the scope side reception unit 121) of the scope 100 waits until a start bit of a packet (a normal time CPU side transmission packet 10 or an initialization time CPU side transmission packet 10P) coming from the image processing device 200 is received (steep 902) at timing when a pulse of the vertical synchronizing signal 301 is detected (step 901) . Upon receipt of the start bit, the scope side control logic circuit 120 checks the sequence flag 11 and the checksum 12 at a stage where it has received the packet from the sequence flag 11 up to the checksum 12 on the start side of the packet (step 903).

If the scope side control logic circuit 120 determines, based on the checksum 12, that the packet has been successfully received, it further determines whether the packet is either a packet for a parameter transfer or a packet at normal times by referencing the initialization/normal time identification flag 11c of the sequence flag 11 (step 904).

If the scope side control logic circuit 120 determines that the packet is the normal time CPU side transmission packet 10, it receives the whole of the data at and after the checksum 12 (step 905) . If the checksum 14 of the control/state data 13 indicates that the packet has been successfully received, the scope side reception unit 121 transfers information of the control/state data 13 to the scope control unit 125 and the like (step 907), passes a trigger of a transmission start request of a return packet to the scope side transmission unit 122 (step 914), and enters a state of waiting for the next vertical synchronizing signal 301 (step 915).

If the scope side control logic circuit 120 determines that the received packet is the initialization time CPU side transmission packet 10P that instructs a parameter transfer, it receives the whole of the data such as the succeeding scope initialization information 15 and checksum 14 (step 910), and determines the checksum 14 (step 911).

If the scope side control logic circuit 120 determines that the packet has been successfully received, it passes the scope initialization information 15 to the scope control unit 125, notifies the scope side transmission unit 122 of information of a transfer area of the specific parameters 500 in the nonvolatile memory 150 (the parameter holding unit 123) in preparation for a reply (step 912), passes the trigger of the transmission start request to the scope side transmission unit 122 (step 914), and waits for the next vertical synchronizing signal 301 (step 915).

In contrast, if the scope side control logic circuit 120 determines that the packet has been unsuccessfully received based on the checksum in the above described step 903, 906, or 911, it discards the current packet, instructs the scope side transmission unit 122 of a NAK reply (sets a NAK bit in the ACK/NAK reply flag 11d of the sequence flag 11) (step 913), and executes step 914 and later.

In the meantime, as illustrated in FIG. 7, at timing when the vertical synchronizing signal 301 is detected (step 951), the scope side transmission unit 122 of the scope side control logic circuit 120 waits for the trigger of the transmission request in step 914 on the side of the above described scope side reception unit 121 (step 952), and reads a determination result of the sequence flag 11 from the scope side reception unit 121 upon receipt of the trigger (step 953) .

If the packet is a normal time packet, the scope side reception unit 121 collects and latches scope realtime information 23 of the scope 100 (step 954), generates a normal time scope side transmission packet 20 composed of a sequence flag 21, a checksum 22, scope realtime information 23, and a checksum 24 (step 956), transmits the packet to the CPU side reception unit 232 of the image processing device 200 (step 957), and waits for the next vertical synchronizing signal 301 (step 960).

If the packet is a NAK reply, it is assumed that the NAK bit is set in the ACK/NAK reply flag 11d of the sequence flag 21 in the above described step 956.

In contrast, if it is determined that the packet is the initialization time CPU side transmission packet 10P for a parameter transfer in the above described step 953, the scope side reception unit 121 verifies a transfer area (information passed from the scope side reception unit 121 in the above described step 912) for the specific parameters 500 of the nonvolatile memory 150 (the parameter holding unit 123) (step 958), generates parameter reply information 25 by selecting and reading data of the specific parameters 500 of the corresponding area in the parameter holding unit 123 (step 959), generates an initialization time scope side transmission packet 20P composed of a sequence flag 21, a checksum 22, parameter reply information 25, and a checksum 24 (step 956), transmits the packet to the image processing device 200 (step 957), and waits for the next vertical synchronizing signal 301 (step 960).

If the trigger of the transmission start request has not been received in the above described step 952, the scope side reception unit 121 waits for the next vertical synchronizing signal 301 (step 960).

As illustrated in FIG. 2, in the case of this embodiment, the scope side transmission unit 122 of the scope 100 generates and transmits the normal time scope side transmission packet 20 or the initialization time scope side transmission packet 20P as a reply after the checksum 14 of the normal time CPU side transmission packet 10 or the initialization time CPU side transmission packet 10P has been received from the image processing device 200. However, the normal time scope side transmission packet 20 or the initialization time scope side transmission packet 20P as a reply may start to be generated and transmitted immediately after the sequence flag 11 and the checksum 12 of the normal time CPU side transmission packet 10 or the initialization time CPU side transmission packet 10P has been received.

Additionally, in the case of this embodiment, data lengths of the normal time CPU side transmission packet 10, the normal time scope side transmission packet 20, the initialization time CPU side transmission packet 10P, and the initialization time scope side transmission packet 20P are set so that a transmission/reception of the normal time CPU side transmission packet 10 and the normal time scope side transmission packet 20 as a reply, and that of the initialization time CPU side transmission packet 10P and the initialization time scope side transmission packet 20P as a reply are completed within one cycle of the vertical synchronizing signal 301.

As described above, the endoscope system S1 according to this embodiment is configured so that a structure of a packet returned as a reply from the scope 100 to the image processing device 200 is selected according to a reception result of a setting content of the initialization/normal time identification flag 11c in the sequence flag 11 at the start of a packet that is transmitted from the side of the image processing device 200 to the scope 100. Therefore, transfers of diverse items of information such as an exchange of the control/state data 13 and the scope realtime information 23 at normal times, a transfer of the specific parameters 500 based on the parameter reply information 25 at activation of the scope 100, and the like can be implemented without providing new dedicated communication lines respectively for the information transfers.

Additionally, transmissions/receptions of data of the normal time CPU side transmission packet 10, the normal time scope side transmission packet 20, the initialization time CPU side transmission packet 10P, and the initialization time scope side transmission packet 20P are performed with start-stop synchronization based on the image capturing element driving clock 302 supplied to the image capturing unit 130 at timing of a common vertical synchronizing signal respectively in the scope side control logic circuit 120 and the processor side control logic circuit 230, which are respectively provided in the scope 100 and the image processing device 200. As a result, a data transfer between the scope 100 and the image processing device 200 can be quickly performed without causing a noise in video data transferred from the image capturing unit 130 to the image processing device 200.

Furthermore, the transmission/reception of the scope side control logic circuit 120 of the scope 100 and the processor side control logic circuit 230 of the image processing device 200 is performed from the start of the flows for each vertical synchronizing signal based on a pulse of the vertical synchronizing signal 301 as illustrated in FIGs. 4 to 7. Therefore, a communication can be resumed even if a communication is abnormally frozen in a state of waiting for a start bit, a reception completion and the like due to a noise or the like. As a result, a noise resistance of a communication is improved.

In this way, diverse pieces of data respectively having different attributes and data amounts can be efficiently transmitted in a short time without increasing the number of transmission paths between the scope 100 and the image processing device 200, which configure the endoscope system S1.

Namely, according to the embodiment 1, diverse pieces of data respectively having different attributes and data amounts can be efficiently transmitted in a short time without increasing the number of transmission paths between the scope 100 and the image processing device 200, which configure the endoscope system S1.

Consequently, the length of time needed to transfer the specific parameters 500 from the nonvolatile memory 150 of the scope 100 to the parameter memory 233 of the image processing device 200 is reduced, for example, when the scope 100 is initialized at its activation, and an observed image can be output early by the image processing device 200 owing to an increase in the efficiency of a data transfer of the specific parameters 500 and the like at activation of the scope 100. This improves the operability of the endoscope system S1.

### (Embodiment 2)

Japanese Laid-open Patent Publication No. 2000-284957, the above described conventional technique, discloses a technique of attempting to securely share an operation program by transferring, to an 10 side circuit, a rewrite program and an update program, which are stored in a nonvolatile memory (ROM) in a patient side circuit at initialization of a multi microcomputer system where processors are respectively arranged in the patient side circuit and the 10 side circuit.

This conventional technique, however, does not disclose a route for writing data to the nonvolatile memory within the patient side circuit.

The ROM of the patient side circuit in the endoscope system is also a storage location of data that changes in real time, such as a power-up time of the scope, or the like, and information, such as a facility name or the like, which is specific to the scope and is freely written by a user. Therefore, the route for reading/writing (R/W) data from the 10 side circuit to the nonvolatile memory of the patient side circuit is needed.

Moreover, an EEPROM or a flash ROM is normally used as the nonvolatile memory. However, R/W (especially a write) from/to these memories needs a considerable amount of time.

There is also a technical challenge to reductions in the number of pins as an interface that connects the patient side circuit and the 10 side circuit. Therefore, the 10 side circuit needs to perform read/write operations from/to the nonvolatile memory of the patient side circuit with a fewer number of serial communication lines as occasion demands, and a system that solves these challenges was demanded.

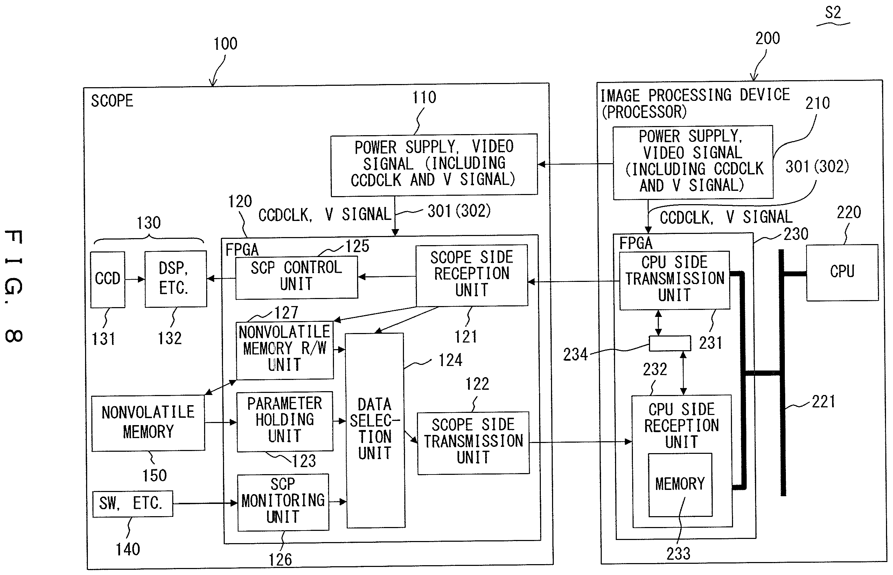

Accordingly, the embodiment 2 refers to a technique of implementing a read/write (R/W) of data such as the specific parameters 500 and the like in the nonvolatile memory 150 of the scope 100 from the side of the image processing device 200, a read/write (R/W) of setting information of the image capturing unit 130, and a read/write (R/W) of an arbitrary register within the scope side control logic circuit 120 with the use of the above described serial communication line 402 and serial communication line 403 during normal running without increasing the number of serial communication lines by adding a configuration illustrated in FIGs. 8 to 12 to be described later to the configuration of the above described embodiment 1.

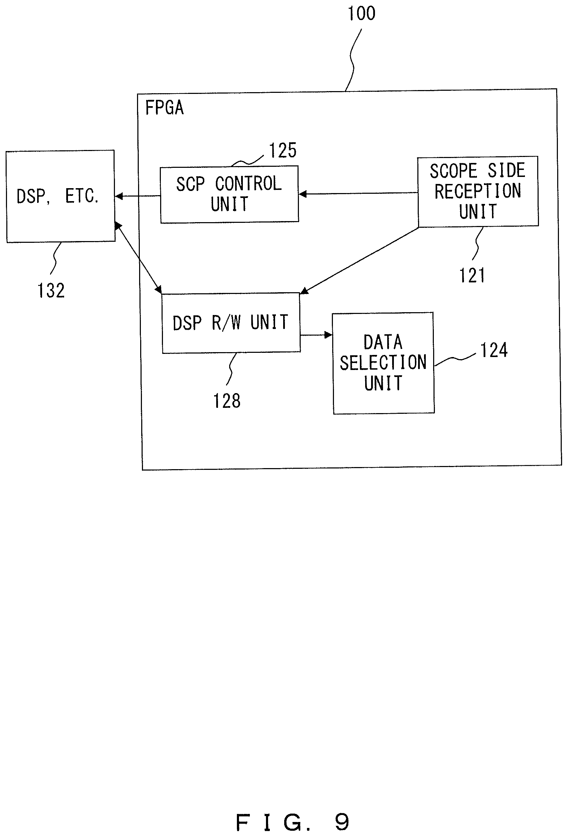

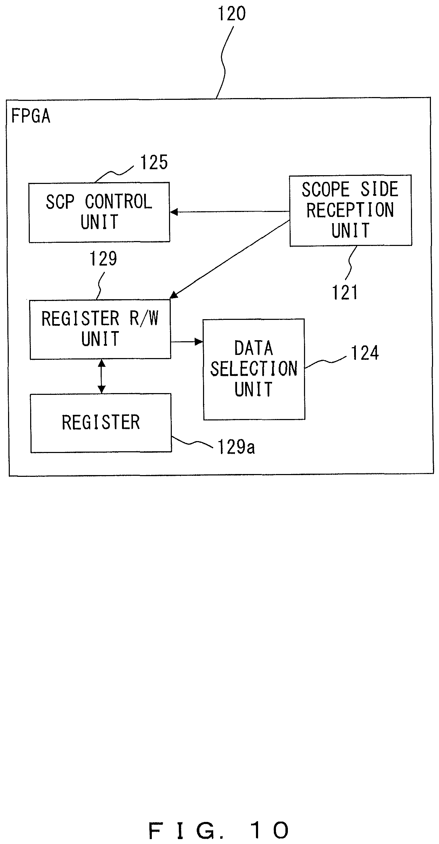

FIG. 8 is a conceptual schematic illustrating a configuration example of an endoscope system for implementing an control method of the endoscope system according to another embodiment of the present invention. FIGs. 9 and 10 illustrate its modification examples.

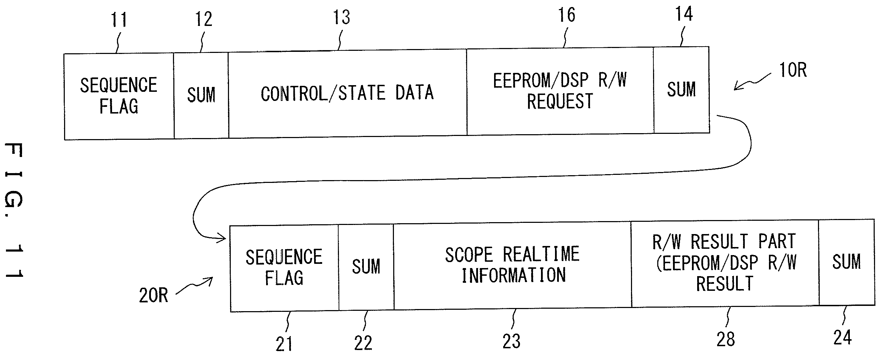

FIG. 11 is a conceptual schematic illustrating an example of a structure of a packet used in the endoscope system for implementing the control method of the endoscope system according to the another of the present invention.

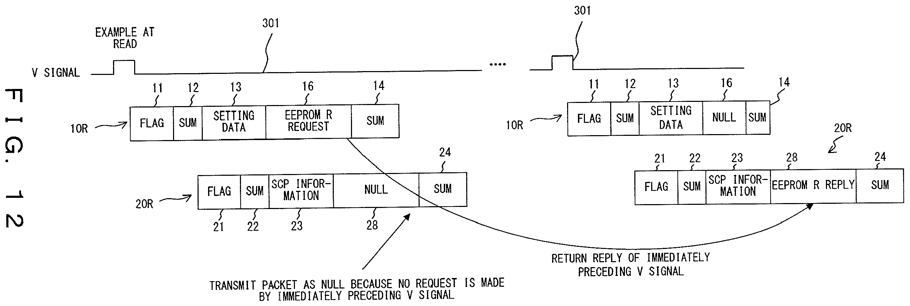

FIG. 12 is a timing chart illustrating one example of actions of the endoscope system for implementing the control method of the endoscope system according to the another embodiment of the present invention.

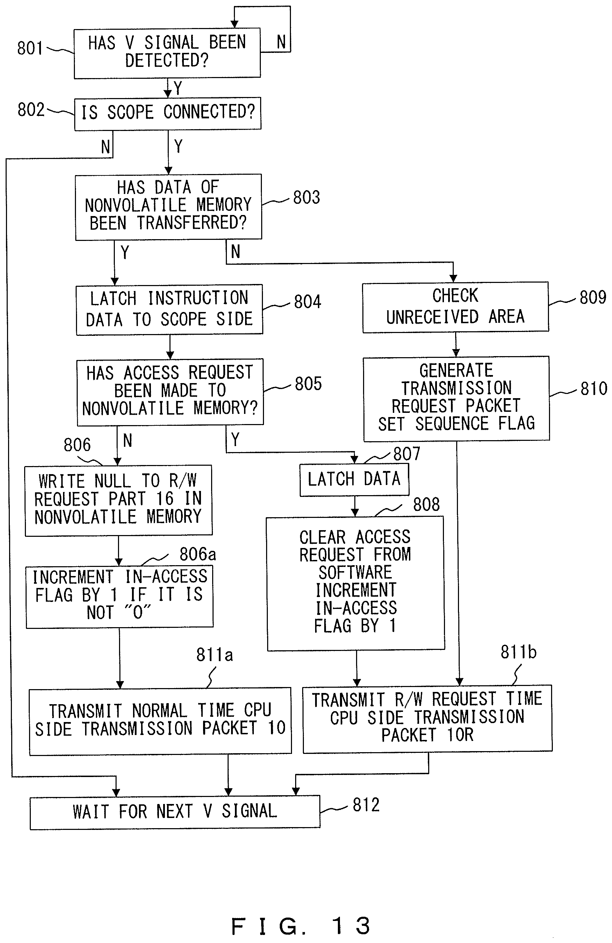

FIG. 13 is a state transition diagram illustrating one example of actions of a hardware circuit for performing a transmission operation on the side of an image processing device in the control method of the endoscope system according to the another embodiment of the present invention.

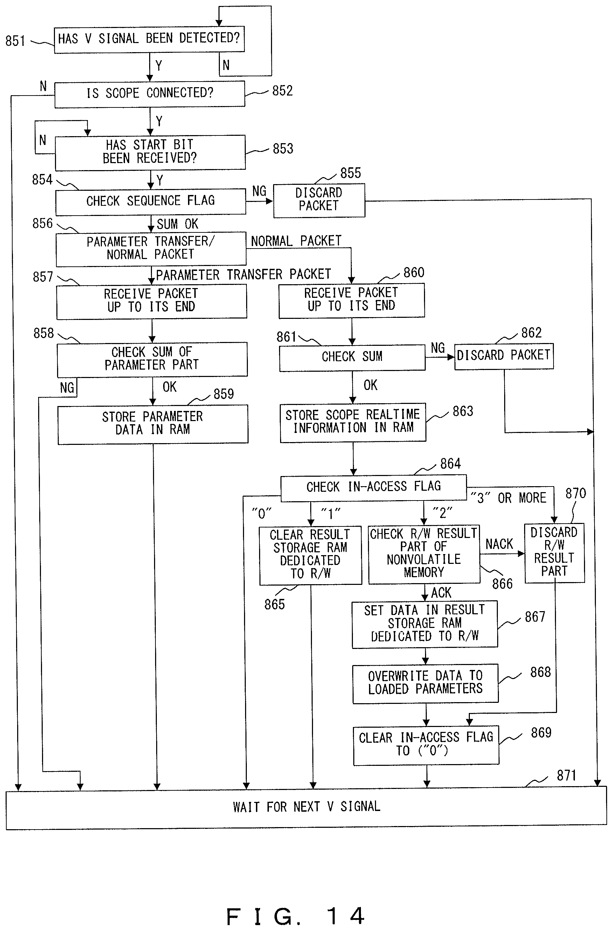

FIG. 14 is a state transition diagram illustrating one example of actions of a hardware circuit for performing a reception operation on the side of the image processing device in the control method of the endoscope system according to the another embodiment of the present invention.

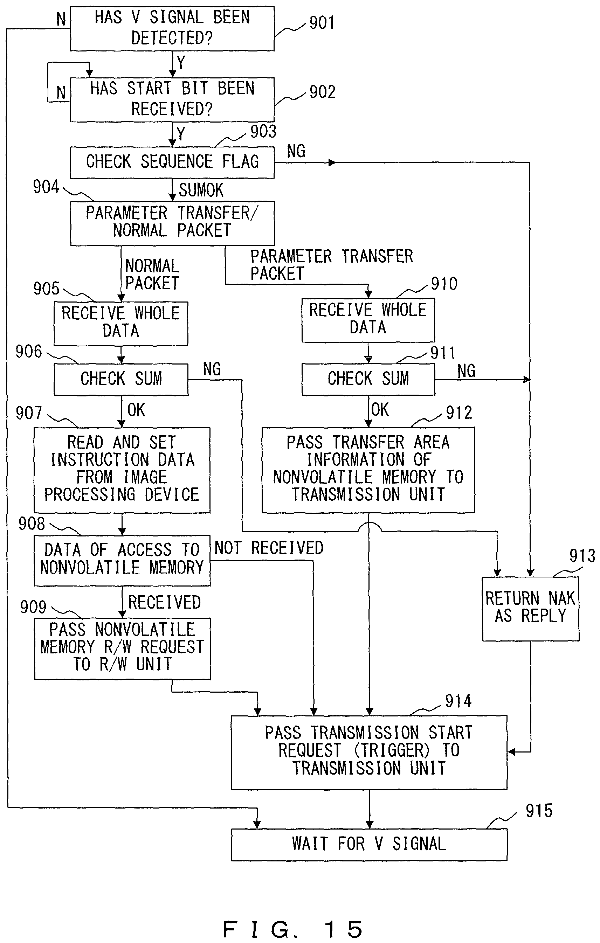

FIG. 15 is a state transition diagram illustrating one example of actions of a hardware circuit for performing a reception operation on the side of a scope in the control method of the endoscope system according to the another embodiment of the present invention.

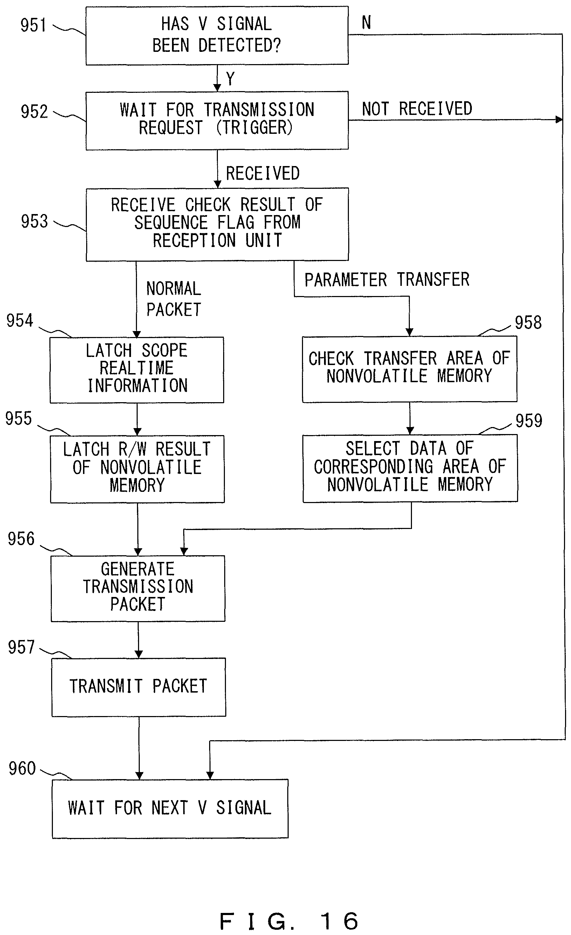

FIG. 16 is a state transition diagram illustrating one example of actions of a hardware circuit for performing a transmission operation on the side of the scope in the control method of the endoscope system according to the another embodiment of the present invention.

In the embodiment 2, the components and the information examples, which are referred to in the above described embodiment 1, are denoted with the same reference numerals, and redundant descriptions are omitted. Also other embodiments to be described later are similar.

In the embodiment 2, an access from the image processing device 200 to the nonvolatile memory 150 of the scope 100 or the like is implemented during normal running by using an R/W request time CPU side transmission packet 10R that is configured by adding an R/W request part 16 as a portion of the above described normal time CPU side transmission packet 10, and an R/W request time scope side transmission packet 20R that is configured by adding an R/W result part 28 as a portion of the above described normal time scope side transmission packet 20 as illustrated in FIG. 11.

Accordingly, in the case of the embodiment 2, a nonvolatile memory R/W unit 127 for implementing an access to the nonvolatile memory 150 by interpreting the R/W request part 16 set in the R/W request time CPU side transmission packet 10R is provided in the scope side control logic circuit 120 of the scope 100 as illustrated in FIG. 8.

Additionally, an in-access flag 234 is provided to manage a state of an access to the nonvolatile memory 150 in the processor side control logic circuit 230 of the image processing device 200.

If an access target is the image capturing element driving unit 132 such as the DSP or the like of the image capturing unit 130, a DSP_R/W unit 128 is provided as a replacement for the nonvolatile memory R/W unit 127 as illustrated in FIG. 9.

Similarly, a register R/W unit 129 is provided as a replacement for the nonvolatile memory R/W unit 127 if the access target is a register 129a of the scope side control logic circuit 120.

Note that the nonvolatile memory R/W unit 127, the DSP_R/W unit 128, and the register R/W unit 129 can be provided together.

Namely, in the embodiment 2, information indicating an R/W request to the nonvolatile memory 150, the image capturing element driving unit 132, and the register 129a of the scope 100 is set in the R/W request part 16 of the R/W request time CPU side transmission packet 10R that is transmitted from the image processing device 200 to the scope 100.

In the R/W request part 16, information indicating an access request, an access destination selection (here, the EEPROM, the DSP, or the register), a start address desired to be accessed at an access destination, a data length, and the like are stored.

Additionally, in the R/W result part 28 provided in the R/W request time scope side transmission packet 20R that is transmitted from the scope 100 to the image processing device 200 in response to the R/W request time CPU side transmission packet 10R, information indicating whether an access is made either successfully or unsuccessfully, and an access result is set.

In the R/W result part 28, for example, information indicating whether an access is made either successfully or unsuccessfully, and read actual data are stored.

If no access request is made from the image processing device 200, the R/W request part 16 is padded with NULL data.

If an access request is made from the image processing device 200, the R/W request time CPU side transmission packet 10R where request data is set in the R/W request part 16 is transmitted from the side of the image processing device 200.

In this embodiment, the R/W request time scope side transmission packet 20R as a reply to an arbitrary R/W request time CPU side transmission packet 10R is returned one cycle after the current vertical synchronizing signal 301 as illustrated in FIG. 12.

Accordingly, the R/W result part 28 of the R/W request time scope side transmission packet 20R returned as a reply to the R/W request time CPU side transmission packet 10R within the same cycle of the vertical synchronizing signal 301 remains NULL because the content of the R/W result part 28 is that one cycle before the current vertical synchronizing signal 301.

Then, in the next cycle, the R/W request part 16 is set to NULL on the side of the image processing device 200, and the scope side control logic circuit 120 (the nonvolatile memory R/W unit 127) of the scope 100 sets the result of the immediately preceding access request in the R/W result part 28, and transmits the packet in the next cycle.

In the transmission/reception of the above described R/W request part 16 and the R/W result part 28 between the image processing device 200 and the scope 100 in the endoscope system S2 according to this embodiment, it is determined that an access is successfully made if a reply (ACK (ACK for the access to the EEPROM) indicating that the access has been successfully made and the same data as data requested to be written are returned from the side of the scope 100 in response to the write request made from the image processing device 200, and the parameter memory 233 is overwritten as will be described later.

Additionally, if no reply is returned (the R/W result part 28 is NULL) in a cycle of the next V signal in response to the access request made from the image processing device 200 to the scope 100, or if the reply indicates a data abnormality (an abnormality of the checksum, a NAK reply of the ACK/NAK reply flag 11d in the sequence flag 21, or the like), the CPU 220 (software) is notified that the access has been unsuccessfully made.

The CPU 220 that has received this notification performs a control for retransmitting the R/W request time CPU side transmission packet 10R to the scope 100 as occasion demands.

Also a handling operation performed when an error occurs at the time of an access made to the DSP (the image capturing element driving unit 132) of FIG. 9 or the register 120a of FIG. 10 other than the EEPROM (the nonvolatile memory 150) illustrated in FIG. 8 is similar.

The transmission/reception operations of the R/W request time CPU side transmission packet 10R including the R/W request part 16, and those of the R/W request time scope side transmission packet 20R including the R/W result part 28 in the endoscope system S2 according to the embodiment 2 are described next with reference to FIGs. 13 to 16.

The basic transmission/reception operations are common to those of the above described embodiment 1 illustrated in FIGs. 4 to 7. Therefore, the common steps are denoted with the common numerals, and redundant descriptions are omitted. A portion different from the first embodiment is described.

Initially, with the transmission operation of the CPU side transmission unit 231 in the processor side control logic circuit 230 of the image processing device 200 illustrated in FIG. 13, whether or not an access request has been made from the CPU 220 (desired software executed by the CPU 220) to the nonvolatile memory 150 is determined (step 805) immediately after the above described step 804 for setting the control/state data 13. If the access request has not been made, the R/W request part 16 is set to NULL (step 806), and the in-access flag 234 is incremented by "1" if it is not "0" (step 806a). Then, the flow proceeds to the above described step 811a.

In contrast, if the access request has been made in step 805, data of the access request is latched (set in the R/W request part 16) (step 807), the access request from the CPU 220 (software) is cleared, and the in-access flag 234 is cleared and incremented by "1" (step 808). Then, the flow proceeds to the above described step 811b, in which the R/W request time CPU side transmission packet 10R is transmitted.

One example of the reception operation of the R/W request time scope side transmission packet 20R in the CPU 220 on the side of the image processing device 200 is described next with reference to FIG. 14.

With this reception operation, the in-access flag 234 is checked after the above described step 863 of FIG. 5 (step 864) .

If the in-access flag 234 is "0", the flow proceeds to step 871.

In contrast, if the in-access flag 234 is "1" as a result of the checking made in the above described step 864, an area (a result storage area dedicated to R/W) of the parameter memory 233 where data read from the nonvolatile memory 150 is stored is cleared (step 865). Then, the flow proceeds to the above described step 871.

Alternatively, if the in-access flag 234 is "2" as a result of the checking made in the above described step 864, whether the access request is made either successfully or unsuccessfully is determined based on ACK/NAK by referencing the R/W result part 28 of the received R/W request time scope side transmission packet 20R (step 866).

If the access request has been successfully made (ACK), data resultant from the access is latched in a buffer of part of the parameter memory 233 (step 867), the data resultant from the access in the R/W result part 28 is overwritten to a corresponding area of the specific parameters 500 loaded in the parameter memory 233 (step 868), and the in-access flag 234 is cleared to "0" (step 869) . Then, the flow proceeds to the above described step 871.

In contrast, if it is determined in step 866 that the access request has been unsuccessfully made (NAK), data of the R/W result part 28 is discarded (step 870). Then, the flow proceeds to step 869, in which the in-access flag 234 is cleared to "0".

The transmission/reception operation on the side of the scope 100 in response to the transmission/reception operation performed on the side of the above described image processing device 200 is described next.

As illustrated in FIG. 15, after reading the control/state data 13 of the R/W request time CPU side transmission packet 10R in the above described step 907, the scope side control logic circuit 120 (the scope side reception unit 121) of the scope 100 reads the succeeding R/W request part 16, and determines whether or not the R/W request part 16 is NULL.

If the request is valid access request information that is not NULL, the information is passed to the nonvolatile memory R/W unit 127 (step 909), and the nonvolatile memory R/W unit 127 is made to access the nonvolatile memory 150. Then, the flow proceeds to the above described step 914.

In contrast, if the R/W request part 16 is NULL in step 908, the flow immediately proceeds to the above described step 914.

An example of the operation of the scope side control logic circuit 120 (the scope side transmission unit 122) of the scope 100 is described next with reference to FIG. 16.

In this case, a result of an access made to the nonvolatile memory 150 is latched after the above described step 954 of FIG. 7, and set in the R/W result part 28 (step 955).

In step 955, if there is an inconsistency in the R/W request part 16 of the R/W request time CPU side transmission packet 10R that has been received by the above described scope side reception unit 121, the R/W result part 28 is set to NULL as an access result in order to prompt the R/W request time CPU side transmission packet 10R to be retransmitted as described above.

A transmission/reception operation of the R/W request time CPU side transmission packet 10R for an access made from the above described image processing device 200 to the nonvolatile memory 150 in the scope 100 is similar if an access target is the DSP (the image capturing element driving unit 132) or the register 129a.

As described above, in the endoscope system S2 according to this embodiment, the CPU side transmission unit 231 and the CPU side reception unit 232 of the image processing device 200 check an access result of the R/W request time scope side transmission packet 20R at timing of a vertical synchronizing signal 301 next to an access request made by the R/W request time CPU side transmission packet 10R, and check a checksum error or whether an access is made either successfully or unsuccessfully. If the access has been successfully made, the access result is stored in a memory such as the parameter memory 233 or the like, and the access is terminated. Namely, as far as no subsequent request is made, the R/W request part 16 is padded with NULL, and the R/W request time CPU side transmission packet 10R is transmitted.

If the access has been unsuccessfully made, it may be retried several times as described above.

In either case, if the access is not made successfully although an access request is made once or several times, the side of the image processing device 200 regards an access as a failure, and terminates the R/W operation.

The side of the scope 100 executes an access request process by the cycle of the next vertical synchronizing signal 301, and transmits an access result to the R/W request time scope side transmission packet 20R at timing of the vertical synchronizing signal 301.

As described above, in the endoscope system S2 according to the embodiment 2, an access can be made from the image processing device 200 to the nonvolatile memory 150, the image capturing element driving unit 132, the register 129a and the like as occasion demands without needing a special communication path other than the transmission/reception paths of the normal time CPU side transmission packet 10 and the normal time scope side transmission packet 20 at normal times, namely, without increasing the number of signal lines.

The endoscope system S2 has advantages such that an image obtained from the scope 100 can be adjusted, for example, by changing from the side of the image processing device 200 an access to the DSP that configures the image capturing element driving unit 132, and the state of a register not illustrated within the scope side control logic circuit 120 for holding control parameters of the scope side control logic circuit 120.

Additionally, in synchronization with the image capturing element driving clock 302 (the CCD clock) for driving the image capturing unit 130, a packet transfer, and operations of the scope side control logic circuit 120 configured with an FPGA or the like, and hardware circuits such as the processor side control logic circuit 230 and the like are performed. Accordingly, there is an advantage such that a result of an access made to the nonvolatile memory 150 or the like can be quickly transmitted/received with the same vertical synchronizing signal 301 as the image capturing unit 130 without concern for a noise.

As a result, in the endoscope system S2, the specific parameters 500 can be partially set or updated with an access to the nonvolatile memory 150, and an output image can be adjusted or the like with an access to the image capturing element driving unit 132 that is configured with the DSP or the like while a normal shooting operation or the like is being performed.

### (Embodiment 3)

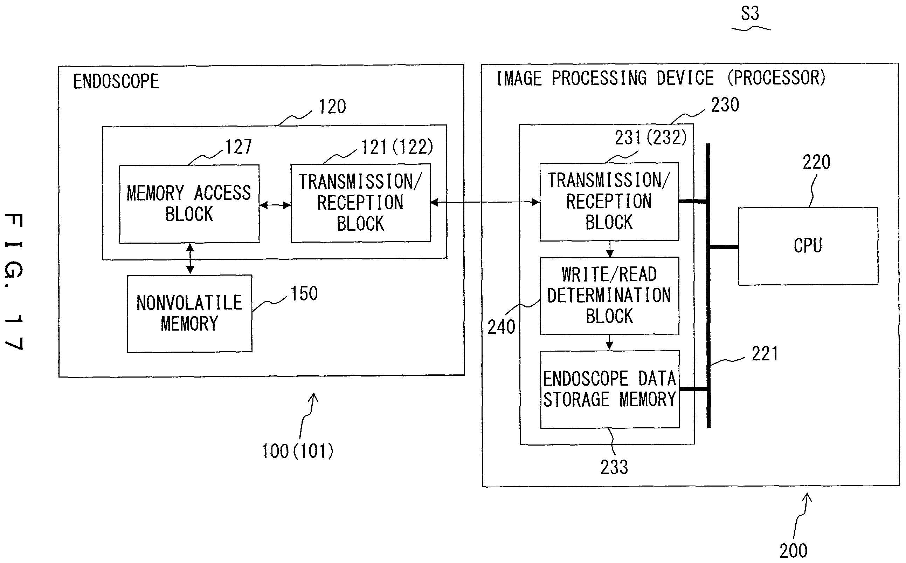

FIG. 17 is a block diagram illustrating one example of a configuration of an endoscope system for implementing a control method of the endoscope system according to a further embodiment of the present invention.

As in the above described embodiment 2, if both the specific parameters 500 stored in the nonvolatile memory 150 of the scope 100 and copied specific parameters 500 in the parameter memory 233 of the image processing device are partially updated during running after the specific parameters 500 are copied from the nonvolatile memory 150 to the parameter memory 233, it is desirable to verify whether or not update data written to the nonvolatile memory 150 and data written to the parameter memory 233 match.

Accordingly, in the embodiment 3, if the same write operation is performed for the side of the nonvolatile memory 150 with the R/W request time CPU side transmission packet 10R at the time of a partial rewrite of the specific parameters 500 in the parameter memory 233, a write result is read into the side of the image processing device 200 by performing a write to the nonvolatile memory 150 and a read as verification from the same area before the parameter memory 233 is actually updated, and the write result is compared with the write data within the processor side control logic circuit 230 of the image processing device. If both of the data match, the parameter memory 233 is actually updated.

As a result, the specific parameters 500 stored in the nonvolatile memory 150 and contents of the specific parameters 500 in the parameter memory 233 can be always made to precisely match.

Accordingly, in the embodiment 3, a read/write determination block 240 is arranged between a transmission/reception block, configured with the CPU side transmission unit 231 and the CPU side reception unit 232, and the parameter memory 233 in the processor side control logic circuit 230 as illustrated in FIG. 17, and the read/write determination block 240 is caused to perform the above described verification operation.

Note that the endoscope system S3 illustrated in FIG. 17 has the same configuration as the above described endoscope system S2 except for the read/write determination block 240, and the endoscope system S3 is simplified and illustrated other than the portion related to the embodiment 3.

An example of actions of the endoscope system S3 according to the embodiment 3 is described below.

All the specific parameters 500 (or the whole of a needed area) in the nonvolatile memory 150 are automatically transferred from the scope 100 to the parameter memory 233 on the side of the image processing device 200 by the scope side control logic circuit 120 and the processor side control logic circuit 230 at activation of the endoscope system S3.

Thereafter, if a request to rewrite the nonvolatile memory 150 occurs in the CPU 220 during normal running, rewrite data and address information are written from the CPU 220 to the transmission/reception block (the CPU side transmission unit 231 and the CPU side reception unit 232), and a transfer request is issued to the transmission/reception block.

The transmission/reception block of the image processing device 200 transfers needed information to the transmission/reception block (the scope side reception unit 121 and the scope side transmission unit 122) on the side of the scope 100 by using the R/W request part 16 of the R/W request time CPU side transmission packet 10R.

The nonvolatile memory R/W unit 127, a memory access block in the scope side control logic circuit 120 of the scope 100, rewrites part of the specific parameters 500 in the nonvolatile memory 150 in response to the write request of the transmission/reception block.

The nonvolatile memory R/W unit 127 again reads the written area of the nonvolatile memory 150 immediately after the rewrite, and transmits a result to the transmission/reception block (the scope side reception unit 121 and the scope side transmission unit 122).

The transmission/reception block (the scope side reception unit 121 and the scope side transmission unit 122) transfers the read result to the transmission/reception block (the CPU side transmission unit 231 and the CPU side reception unit 232) of the image processing device 200 by using the R/W result part 28 of the R/W request time scope side transmission packet 20R.

The read/write determination block 240 of the image processing device 200 receives the read result from the transmission/reception block (the CPU side transmission unit 231 and the CPU side reception unit 232), and determines whether or not the result is the same as the write data.

The read/write determination block 240 rewrites the corresponding area of the parameter memory 233 only if the result is the same as the write data.

As described above, with the endoscope system S3 according to the embodiment 3, no inconsistency occurs during running between the specific parameters 500 stored in the nonvolatile memory 150 or the like on the side of the scope 100 and the specific parameters 500 stored in the parameter memory 233 or the like of the image processing device 200, whereby stable and precise operations can be performed in the whole of the endoscope system S3.

The aforementioned embodiments 2 and 3, described separately for the sake of convenience in the above description, can be combined. In that case, for example, the following effects can be further produced.

Namely, if the CPU 220 (software) of the image processing device 200 arbitrarily updates (R/W) the specific parameters 500 stored in the parameter memory 233 during running as occasion demands, consistency needs to be maintained by updating also a corresponding portion of the specific parameters 500 stored in the nonvolatile memory 150 on the side of the scope 100.

In that case, for an access to the nonvolatile memory 150, it is needed to perform a write to the nonvolatile memory 150, verification by a read from the nonvolatile memory 150, a rewrite to a corresponding portion of the specific parameters 500 that are transferred to the parameter memory 233 within the image processing device 200 after the verification.

In this case, in the system according to the above described embodiment 2 that needs two cycles of the vertical synchronizing signal 301 for an access made from the image processing device 200 to the nonvolatile memory 150 of the scope 100, only a data transfer between the scope 100 and the image processing device 200 needs 4 cycles of the vertical synchronizing signal 301 when the above described access accompanied by verification is made.

Therefore, the access accompanied by verification, which is made from the image processing device 200 to the scope 100, is completed in two cycles of the vertical synchronizing signal 301 similarly to a normal access by combining the operations of the embodiment 3 and the above described embodiment 2 as follows.

Namely, on the side of the scope 100, a read from the same written address area is performed immediately after a write when a partial update, requested from the image processing device 200, of the specific parameters 500 is made in the nonvolatile memory 150, and the read data is simultaneously transferred as verification data in a reply cycle of the next vertical synchronizing signal 301 to the image processing device 200.

On the side of the image processing device 200, a result of the partial write to the nonvolatile memory 150 of the scope 100 is immediately returned as a reply. Therefore, a partial update of the specific parameters 500 accompanied by verification can be implemented by comparing the returned verification data with locally held data.

By combining the embodiment 2 and the embodiment 3 as described above, a response of the entire endoscope system can be improved also at the time of a partial update of the specific parameters 500 accompanied by verification.

### (Embodiment 4)

An endoscope including an actuator mechanism using SMA (Shape Memory Alloys) is proposed in a conventional endoscope system. The actuator mechanism is composed of two CPUs such as a first CPU for mainly controlling an SMA actuator, and a second CPU as a main CPU for controlling operations of the whole of the endoscope.

Such a configuration has the following problems in a stable control of the SMA actuator.

Firstly, the SMA actuator needs to be warmed up for a certain period in order to stably operate the SMA actuator with the above described mechanism after the first CPU is activated. However, since the first CPU for controlling the SMA actuator is activated by the second CPU, there is a technical problem such that activation, which needs a considerable amount of time, by the second CPU is to be waited so as to warm up the SMA actuator.

Secondly, it is needed to allow the SMA actuator to operate immediately after the activation of the second CPU as a main CPU. With the conventional configuration, however, the first CPU gets started after the second CPU is activated. Accordingly, there is a technical problem such that a considerable amount of time is needed until the SMA actuator can operate.

Thirdly, whether or not the SMA actuator is included depends on a connected scope. Accordingly, there is a technical problem such that the first CPU needs to be activated and deactivated according to a type of the connected scope.

Accordingly, in the embodiment 4, data for controlling the SMA actuator is stored in a nonvolatile memory included in the endoscope, the data is read by hardware after the endoscope is powered up, and activation, deactivation, and a setting are reflected on the first CPU for controlling the SMA actuator not by software but by hardware. As a result, the endoscope system S4 that has high usability and can stably control the SMA actuator immediately after the second CPU as a main CPU is activated is provided.

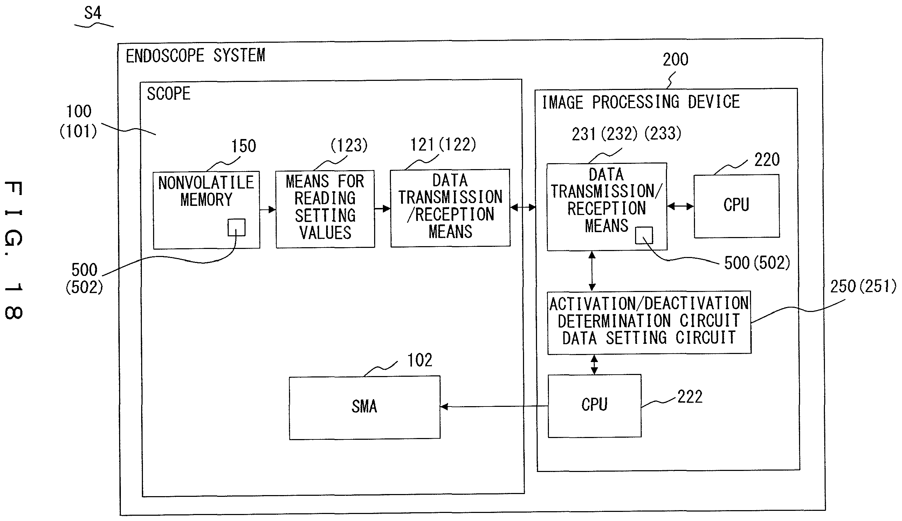

FIG. 18 is a block diagram illustrating one example of a configuration of an endoscope system for implementing a control method of the endoscope system according to a still further embodiment of the present invention.

The endoscope system S4 according to this embodiment illustrated in FIG. 18 is similar to that of the above described embodiment 2. Components common to those of the embodiment 2 are denoted with the common numerals, and only a portion needed to describe the embodiment 4 is illustrated.

As illustrated in FIG. 18, a shape memory alloy actuator 102 is provided on the side of the endoscope 101 having the scope 100 in the endoscope system S4 according to this embodiment.

Additionally, a second CPU 222 for controlling the shape memory alloy actuator 102 is provided in addition to the above described CPU 220 for controlling the whole of the endoscope system S4 on the side of the image processing device 200.

Furthermore, a CPU activation/deactivation determination circuit 250 and a data setting circuit 251 are provided as part of the scope side control logic circuit 120 of the image processing device 200. The CPU activation/deactivation determination circuit 250 and the data setting circuit 251 have a function of initializing the second CPU 222 with no intervention of the CPU 220 with the use of the specific parameters 500 read into the parameter memory 233 with a transmission/reception of the initialization time CPU side transmission packet 10P and the initialization time scope side transmission packet 20P by the CPU side transmission unit 231 and the CPU side reception unit 232, which are configured as part of the hardware circuit of the scope side control logic circuit 120 as referred to in the above described embodiments.

Actions of the endoscope system S4 according to the embodiment 4 are described below.

Shape memory alloy control means setting values 502 are stored as part of the specific parameters 500 in the nonvolatile memory 150 included in the scope 100 of the endoscope 101.

The specific parameters 500 (the shape memory alloy control means setting values 502) are read from the nonvolatile memory 150 by the parameter holding unit 123, and the read shape memory alloy control means setting values 502 are transmitted to the parameter memory 233 of the image processing device 200 by the scope side reception unit 121 and the scope side transmission unit 122.

The shape memory alloy control means setting values 502 received by the parameter memory 233 are transmitted to the CPU activation/deactivation determination circuit 250.

The CPU activation/deactivation determination circuit 250 of the scope side control logic circuit 120 activates or deactivates the second CPU 222 (in the case of the scope 100 that does not include the shape memory alloy actuator 102) with no intervention of the CPU 220 based on data of the transmitted shape memory alloy control means setting values 502. When the second CPU 222 is activated, it is initialized by the data setting circuit 251 based on settings of the above described shape memory alloy control means setting values.

After the second CPU 222 has been activated, the shape memory alloy actuator 102 is warmed up based on the data of the shape memory alloy control means setting values 502 set in the parameter memory 233. Thereafter, the shape memory alloy actuator 102 is controlled during normal running based on an instruction from the CPU 220 or the like.

As described above, with the endoscope system S4 according to the embodiment 4, the following effects can be produced.
(1) The second CPU 222 for controlling the shape memory alloy actuator 102 can be activated with no intervention of the CPU 220, and the length of time needed until the shape memory alloy actuator 102 starts to stably operate after the activation of the endoscope system S4 can be reduced.
(2) The shape memory alloy actuator 102 can start to be early warmed up by the second CPU 222 that is early activated irrespective of whether or not the CPU 220 has been activated, and a control of the shape memory alloy actuator 102 can be started immediately after the activation of the CPU 220 as a main CPU.
(3) If the scope 100 that does not include the shape memory alloy actuator 102 is connected to the image processing device 200, the second CPU 222 can be deactivated by the CPU activation/deactivation determination circuit 250 with no intervention of the CPU 220, leading to a power saving of the endoscope system S4.

The present invention is not limited to the configurations referred to in the above described embodiments, and can be diversely modified.

### (Notes)

1. An endoscope system where specific parameters of a scope are stored within the scope and data of the specific parameters is transferred with a start-stop synchronous communication for each vertical synchronizing signal (V signal) with reference to an operation clock (CCDCLK) of an image capturing element, comprising:
   storage means for storing the specific parameters of the scope;
   parameter holding means, provided within the scope, for reading and holding the data of all the specific parameters of the scope at power-up;
   scope state monitoring means for obtaining information of a scope switch and the like within the scope;
   scope operation control means for dynamically switching and operating an electronic shutter or the like within the scope;
   data transmission means 1 for transmitting data from a processor to the scope;
   data reception means 1, provided within the scope, for receiving the transmitted data;
   data transmission means 2, provided within the scope, for transmitting data within the scope after verifying communication determination data from a start up to a predetermined bit of the data transmitted from the processor to the scope;
   data selection means, provided within the scope, for changing a type of returned data and a packet structure depending on the received communication determination data; and
   data reception means 2, provided within the processor, for receiving the data transmitted from the scope to the processor, the data reception means 2 having a memory for storing the received data, wherein
   at least two types of electronic text formats such as a data format for transferring parameters to the processor, a normal data format for transferring information of the electronic shutter and the scope switch are possessed, and
   after powered up, the processor receives all the specific parameters or some needed parameters of the specific parameters by selecting the format for transferring parameters, stores the received specific parameters in a memory or a register within the reception means, and monitors and operates the scope in the normal format.
2. An endoscope system where specific parameters of a scope are stored within the scope and data of the specific parameters is transferred with a start-stop synchronous communication for each vertical synchronizing signal (V signal) with reference to an operation clock (CCDCLK) of an image capturing element, comprising:
   storage means for storing the specific parameters of the scope;
   data transmission means 1 for transmitting data from a processor to the scope;
   data reception means 1, provided within the scope, for receiving the transmitted data;
   data transmission means 2 for transmitting data within the scope after verifying communication determination data from a start up to a predetermined bit of the data transmitted from the processor to the scope;
   data selection means, provided within the scope, for changing a structure of a returned packet depending on the received communication determination data; and
   reception means 2, provided within the processor, for receiving the data transmitted from the scope to the processor, the reception means 2 having a memory for storing part of the received data, wherein
   at least two types of electronic text formats such as a data format for transferring parameters to the processor, and a normal data format for transferring information of an electronic shutter and a scope switch are possessed,
   an area for data of an access to a nonvolatile memory is provided in the normal data format, and
   a read/write (R/W) result is transmitted from the scope to an image processing device one cycle after a current vertical synchronizing signal, and the result is stored in the memory within the reception means 2.
3. An endoscope system having an endoscope including a nonvolatile memory for storing data needed for the system, the endoscope system transferring part or a whole of data of the nonvolatile memory to an image processing device, which holds the data, only with hardware when the endoscope is connected, wherein
   corresponding data of the held data is rewritten only if data that is written by reading a same area can be properly written after the data within the nonvolatile memory is rewritten.
4. An endoscope system having two CPUs such as a first CPU for mainly controlling an actuator made of a shape memory alloy, and a second CPU as a main CPU, comprising:
   an endoscope including a nonvolatile memory and an actuator;
   means for reading a setting value of actuator control means from the nonvolatile memory at activation of the system;
   means for transmitting/receiving the read setting value;
   means for transmitting/receiving the setting value;
   a circuit for activating the first CPU based on the setting value; and
   a circuit for setting the setting value of the actuator control means in the first CPU, wherein
   after powered up, activation, deactivation and a setting are reflected on the first CPU for controlling the actuator by hardware such as an FPGA or the like, which configures the means and the circuits, with no intervention of software of the second CPU, so that the actuator can be stably controlled immediately after the second CPU is activated.
5. An endoscope system, comprising:
   an endoscope including a nonvolatile memory;
   image capturing means, composed of an image capturing element and an image signal processing unit, for outputting and displaying an image on a monitor by connecting the endoscope; and
   a hardware circuit, such as an FPGA or the like, for storing the setting value of the image capturing means in the nonvolatile memory, and for reading the setting value from the nonvolatile memory and setting the read setting value in the image capturing means with no intervention of software at activation of the system.
6. The endoscope system according to note 5, wherein
   an interface to the image capturing means is a serial communication, and data stored in the nonvolatile memory is serial communication data.
7. The endoscope system according to note 5 or 6, wherein
   a communication speed is automatically corrected for each type of the image capturing means such as a CCD or the like.

### Description of the Symbols

- 10: normal time CPU side transmission packet
- 10P: initialization time CPU side transmission packet
- 10R: R/W request time CPU side transmission packet
- 11: sequence flag
- 11b: communication version flag
- 11c: initialization/normal time identification flag
- 11d: ACK/NAK reply flag
- 12: checksum
- 13: control/state data
- 14: checksum
- 15: scope initialization information
- 16: R/W request part
- 20: normal time scope side transmission packet
- 20P: initialization time scope side transmission packet
- 20R: R/W request time scope side transmission packet
- 21: sequence flag
- 22: checksum
- 23: scope realtime information
- 24: checksum
- 25: parameter reply information
- 26: parameter block information
- 27: parameter values
- 28: R/W result part
- 100: scope
- 101: endoscope
- 102: shape memory alloy actuator
- 110: power/clock supply circuit
- 120: scope side control logic circuit
- 120a: register
- 121: scope side reception unit
- 122: scope side transmission unit
- 123: parameter holding unit
- 124: data selection unit
- 125: scope control unit
- 126: scope monitoring unit
- 127: nonvolatile memory R/W unit
- 128: DSP_R/W unit
- 129: register R/W unit
- 129a: register
- 130: image capturing unit
- 131: image capturing element
- 132: image capturing element driving unit
- 140: scope operation unit
- 150: nonvolatile memory
- 200: image processing device
- 210: power/clock supply circuit
- 220: CPU
- 221: bus
- 222: second CPU
- 230: processor side control logic circuit
- 231: CPU side transmission unit
- 232: CPU side reception unit
- 233: parameter memory
- 234: in-access flag
- 240: read/write determination block
- 250: CPU activation/deactivation determination circuit
- 251: data setting circuit
- 301: vertical synchronizing signal
- 302: image capturing element driving clock
- 400: parameters
- 401: interface line
- 402: serial communication line
- 403: serial communication line
- 500: parameters
- 502: shape memory alloy control means setting value
- S1: endoscope system S1
- S2: endoscope system S2
- S3: endoscope system S3
- S4: endoscope system S4

## Claims

1. An endoscope system, comprising:
a scope unit (100) having an image capturing unit (130) for observing a subject;
a nonvolatile memory (150), provided in the scope unit (100), for storing specific parameters (500) of the scope unit (100);
an image processing device (200) in which at least one of scope units is configured to be freely attachable/detachable and which can process a video signal obtained by the image capturing unit (130) of the connected scope unit;
a first data transmission unit (231), provided in the image processing device (200), for transmitting, to the scope unit (100), a first packet including a signal for requesting the specific parameters (500) or realtime information indicating an operation state of the scope unit (100);
a first data reception unit (232), provided in the image processing device (200), for receiving a second packet that is transmitted from the scope unit (100) based on the first packet and includes the specific parameters (500) or the realtime information;
a second data reception unit (121), provided in the scope unit (100), for receiving the first packet from the image processing device (200); and
a second data transmission unit (122) for transmitting the second packet in the data format selected by the data selection unit to the first data reception unit (232), wherein
the first packet including identification information for requesting the second packet in the first data format includes moving picture standard specification information for specifying a video standard of the image capturing unit (130), and
the first packet including identification information for requesting the second packet in the second data format includes running control information of the scope unit (100),
**characterized in that**
the scope unit (100) further comprises a data selection unit (124) for selecting whether the second packet is transferred either in a first data format for transferring the specific parameters (500) to the image processing device (200) or in a second data format for transferring the realtime information to the image processing device (200) based on the signal that is included in the first packet received by the second data reception unit (12); and
the image processing device (200) further comprises
a parameter storage unit (233) for storing the specific parameters (500) transferred from the nonvolatile memory (150) of the scope unit (100) by the second packet in the first data format, and
a parameter update determination unit (240) for making a partial change of the specific parameters (500) in the parameter storage unit (233) only in a case where the access result information is correct when the partial change of the specific parameters (500) occurs in the parameter storage unit (233), wherein
partial access request information for the specific parameters (500) of the nonvolatile memory (150) is set in addition to the running control information in the first packet including the identification information for requesting the second packet in the second data format, and
access result information for replying to the partial access request information is set in the second packet in the second data format.

2. The endoscope system according to any one of claim 1, wherein
a transmission/reception of the first packet and the second packet between the image processing device (200) and the scope unit (100) is started at timing of a vertical synchronizing signal of the image capturing unit (130), and
a transfer of the first packet and the second packet is made in a start-stop synchronous communication with reference to an operation clock of the image capturing unit (130).

3. The endoscope system according to claim 1, wherein the second packet in the second data format in which the access result information for replying to the partial access request information is set is transmitted from the scope unit (100) to the image processing device (200) with a one-cycle delay of a vertical synchronizing signal of the image capturing unit (130).

4. An operation method of an endoscope system having a scope unit (100) including an image capturing unit (130) for observing a subject, a nonvolatile memory (150), provided in the scope unit (100), for storing specific parameters (500) of the scope unit (100), and an image processing device (200) in which at least one of scope units is configured to be freely attachable/detachable and which can process a video signal obtained by the image capturing unit (130) of the connected scope unit, the method comprising:
a first data transmission step of transmitting, by the image processing device (200), to the scope unit (100) a first packet including a signal for requesting the specific parameters (500) or realtime information indicating an operation state of the scope unit (100);
a data reception step of receiving, by the scope unit (100), the first packet from the image processing device (200);
a data transmission step of transmitting, by the scope unit (100), the second packet selected by the data selection step to the image processing device (200); and
a data reception step of receiving, by the image processing device (200), the second packet that is transmitted from the scope unit (100) based on the first packet, wherein
the first packet including identification information for requesting the second packet in the first data format includes moving picture standard specification information for specifying a video standard of the image capturing unit (130), and
the first packet including identification information for requesting the second packet in the second data format includes running control information of the scope unit (100),
**characterized by**
a data selection step of selecting, by the scope unit (100), whether the second packet is transferred either in a first data format for transferring the specific parameters (500) to the image processing device (200) when the specific parameters (500) are transmitted or in a second data format for transferring the realtime information to the image processing device when the realtime information is transmitted based on the signal that is included in the first packet received by the data reception step in the scope unit (100), wherein
partial access request information for the specific parameters (500) of the nonvolatile memory (150) is set in addition to the running control information in the first packet including the identification information for requesting the second packet in the second data format,
access result information for replying to the partial access request information is set in the second packet in the second data format, and
when a partial change of the specific parameters (500) occurs in a parameter storage unit, provided in the image processing device (200), for storing the specific parameters (500) transferred from the nonvolatile memory (150) of the scope unit (100) by the second packet in the first data format, the partial change of the specific parameters (500) is made in the parameter storage unit only in a case where the access result information is correct.

5. The operation method of the endoscope system according to claim 4, wherein
a transmission/reception of the first packet and the second packet between the image processing device (200) and the scope unit (100) is started at timing of a vertical synchronizing signal of the image capturing unit (130), and
a transfer of the first packet and the second packet is made in a start-stop synchronous communication with reference to an operation clock of the image capturing unit (130).

6. The operation method of the endoscope system according to claim 4, wherein
the second packet in the second data format in which the access result information for replying to the partial access request information is set is transmitted from the scope unit (100) to the image processing device (200) with a one-cycle delay of a vertical synchronizing signal of the image capturing unit (130).

## Patentansprüche

1. Endoskopsystem, umfassend:
eine Endoskopeinheit (100), die eine Bildaufnahmeeinheit (130) zum Beobachten eines Subjekts aufweist;
einen nicht flüchtigen Speicher (150), der in der Endoskopeinheit (100) bereitgestellt wird, um spezifische Parameter (500) der Endoskopeinheit (100) zu speichern;
eine Bildverarbeitungsvorrichtung (200), in der mindestens eine der Endoskopeinheiten konfiguriert ist, um frei anbringbar/abnehmbar zu sein, und die ein Videosignal verarbeiten kann, das durch die Bildaufnahmeeinheit (130) der angeschlossenen Endoskopeinheit erzielt wird;
eine erste Datensendeeinheit (231), die in der Bildverarbeitungsvorrichtung (200) bereitgestellt wird, um an die Endoskopeinheit (100) ein erstes Paket zu senden, das ein Signal zum Anfragen der spezifischen Parameter (500) oder von Echtzeitinformationen, die einen Betriebszustand der Endoskopeinheit (100) angeben, umfasst;
eine erste Datenempfangseinheit (232), die in der Bildverarbeitungsvorrichtung (200) bereitgestellt wird, um ein zweites Paket zu empfangen, das von der Endoskopeinheit (100) basierend auf dem ersten Paket gesendet wird und die spezifischen Parameter (500) oder die Echtzeitinformationen umfasst;
eine zweite Datenempfangseinheit (121), die in der Endoskopeinheit (100) bereitgestellt wird, um das erste Paket von der Bildverarbeitungsvorrichtung (200) zu empfangen; und
eine zweite Datensendeeinheit (122), um das zweite Paket in dem Datenformat, das von der Datenauswahleinheit ausgewählt wird, an die erste Datenempfangseinheit (232) zu senden, wobei
das erste Paket, das Identifizierungsinformationen umfasst, um das zweite Paket in dem ersten Datenformat anzufragen, Informationen über eine Normvorgabe für Bewegtbilder umfasst, um eine Videonorm der Bildaufnahmeeinheit (130) vorzugeben, und
das erste Paket, das Identifizierungsinformationen umfasst, um das zweite Paket in dem zweiten Datenformat anzufragen, Informationen über die Laufregelung der Endoskopeinheit (100) umfasst,
**dadurch gekennzeichnet, dass**
die Endoskopeinheit (100) ferner eine Datenauswahleinheit (124) umfasst, um auszuwählen, ob das zweite Paket entweder in einem ersten Datenformat zum Übertragen der spezifischen Parameter (500) an die Bildverarbeitungsvorrichtung (200) oder in einem zweiten Datenformat zum Übertragen der Echtzeitinformationen an die Bildverarbeitungsvorrichtung (200) basierend auf dem Signal, das in dem ersten Paket enthalten ist, das von der zweiten Datenempfangseinheit (12) empfangen wird, übertragen wird; und
die Bildverarbeitungsvorrichtung (200) ferner umfasst:
eine Parameterspeichereinheit (233), um die spezifischen Parameter (500), die von dem nicht flüchtigen Speicher (150) der Endoskopeinheit (100) durch das zweite Paket in dem ersten Datenformat übertragen werden, zu speichern, und
eine Einheit (240) zum Bestimmen einer Parameteraktualisierung, um eine Teiländerung der spezifischen Parameter (500) in der Parameterspeichereinheit (233) nur für den Fall vorzunehmen, dass die Zugriffsergebnisinformationen richtig sind, wenn die Teiländerung der spezifischen Parameter (500) in der Parameterspeichereinheit (233) erfolgt, wobei
Teilzugriffsanfrageinformationen für die spezifischen Parameter (500) des nicht flüchtigen Speichers (150) zusätzlich zu den Informationen über die Laufregelung in dem ersten Paket eingestellt sind, das die Identifizierungsinformationen zum Anfragen des zweiten Pakets in dem zweiten Datenformat umfasst, und
Zugriffsergebnisinformationen zum Antworten auf die Teilzugriffsanfrageinformationen in dem zweiten Paket in dem zweiten Datenformat eingestellt sind.

2. Endoskopsystem nach Anspruch 1, wobei
ein Senden/Empfangen des ersten Pakets und des zweiten Pakets zwischen der Bildverarbeitungsvorrichtung (200) und der Endoskopeinheit (100) an einer Zeiteinstellung eines vertikalen Synchronisationssignals der Bildaufnahmeeinheit (130) beginnt, und
eine Übertragung des ersten Pakets und des zweiten Pakets in einer synchronen Start-Stopp-Kommunikation mit Bezug auf einen Betriebstakt der Bildaufnahmeeinheit (130) erfolgt.

3. Endoskopsystem nach Anspruch 1, wobei das zweite Paket in dem zweiten Datenformat, in dem die Zugriffsergebnisinformationen zum Antworten auf die Teilzugriffsanfrageinformationen eingestellt sind, von der Endoskopeinheit (100) an die Bildverarbeitungsvorrichtung (200) mit einer Verzögerung eines Zyklus eines vertikalen Synchronisationssignals der Bildaufnahmeeinheit (130) gesendet werden.

4. Betriebsverfahren eines Endoskopsystems, das eine Endoskopeinheit (100), die eine Bildaufnahmeeinheit (130) zum Beobachten eines Subjekts, einen nicht flüchtigen Speicher (150), der in der Endoskopeinheit (100) bereitgestellt wird, um spezifische Parameter (500) der Endoskopeinheit (100) zu speichern, und eine Bildverarbeitungsvorrichtung (200), in der mindestens eine der Endoskopeinheiten konfiguriert ist, um frei anbringbar/abnehmbar zu sein, und die ein Videosignal verarbeiten kann, das durch die Bildaufnahmeeinheit (130) der angeschlossenen Endoskopeinheit erzielt wird, aufweist, wobei das Verfahren die Schritte umfasst:
einen ersten Datensendeschritt des Sendens durch die Bildverarbeitungsvorrichtung (200) an die Endoskopeinheit (100) eines ersten Pakets, das ein Signal umfasst, um die spezifischen Parameter (500) oder Echtzeitinformationen, die einen Betriebszustand der Endoskopeinheit (100) angeben, anzufragen;
einen Datenempfangsschritt des Empfangens durch die Endoskopeinheit (100) des ersten Pakets von der Bildverarbeitungsvorrichtung (200);
einen Datensendeschritt des Sendens durch die Endoskopeinheit (100) des zweiten Pakets, das durch den Datenauswahlschritt ausgewählt wird, an die Bildverarbeitungsvorrichtung (200); und
einen Datenempfangsschritt des Empfangens durch die Bildverarbeitungsvorrichtung (200) des zweiten Pakets, das von der Endoskopeinheit (100) basierend auf dem ersten Paket gesendet wird, wobei
das erste Paket, das Identifizierungsinformationen umfasst, um das zweite Paket in dem ersten Datenformat anzufragen, Informationen über Normvorgaben für Bewegtbilder umfasst, um eine Videonorm der Bildaufnahmeeinheit (130) vorzugeben, und
das erste Paket, das Identifizierungsinformationen zum Anfragen des zweiten Pakets in dem zweiten Datenformat umfasst, Informationen über die Laufregelung der Endoskopeinheit (100) umfasst,
**gekennzeichnet durch**
einen Datenauswahlschritt des Auswählens durch die Endoskopeinheit (100), ob das zweite Paket entweder in einem ersten Datenformat zum Übertragen der spezifischen Parameter (500) an die Bildverarbeitungsvorrichtung (200) übertragen wird, wenn die spezifischen Parameter (500) gesendet werden, oder in einem zweiten Datenformat zum Übertragen der Echtzeitinformationen an die Bildverarbeitungsvorrichtung übertragen wird, wenn die Echtzeitinformationen basierend auf dem Signal gesendet werden, das in dem ersten Paket enthalten ist, das in dem Datenempfangsschritt in der Endoskopeinheit (100) empfangen wird, wobei
Teilzugriffsanfrageinformationen für die spezifischen Parameter (500) des nicht flüchtigen Speichers (150) zusätzlich zu den Informationen über die Laufregelung in dem ersten Paket eingestellt sind, das die Identifizierungsinformationen zum Anfragen des zweiten Pakets in dem zweiten Datenformat umfasst,
Zugriffergebnisinformationen zum Antworten auf die Teilzugriffsanfrageinformationen in dem zweiten Paket in dem zweiten Datenformat enthalten sind, und
wenn eine Teiländerung der spezifischen Parameter (500) in einer Parameterspeichereinheit erfolgt, die in der Bildverarbeitungsvorrichtung (200) bereitgestellt wird, um die spezifischen Parameter (500) zu speichern, die von dem nicht flüchtigen Speicher (150) der Endoskopeinheit (100) durch das zweite Paket in dem ersten Datenformat übertragen werden, die Teiländerung der spezifischen Parameter (500) in der Parameterspeichereinheit nur für den Fall erfolgt, dass die Zugriffsergebnisinformationen richtig sind.

5. Betriebsverfahren des Endoskopsystems nach Anspruch 4, wobei
ein Senden/Empfangen des ersten Pakets und des zweiten Pakets zwischen der Bildverarbeitungsvorrichtung (200) und der Endoskopeinheit (100) an einer Zeiteinstellung eines vertikalen Synchronisationssignals der Bildaufnahmeeinheit (130) beginnt, und
eine Übertragung des ersten Pakets und des zweiten Pakets in einer synchronen Start-Stopp-Kommunikation mit Bezug auf einen Betriebstakt der Bildaufnahmeeinheit (130) erfolgt.

6. Betriebsverfahren des Endoskopsystems nach Anspruch 4, wobei
das zweite Paket in dem zweiten Datenformat, in dem die Zugriffsergebnisinformationen zum Antworten auf die Teilzugriffsanfrageinformationen eingestellt sind, von der Endoskopeinheit (100) an die Bildverarbeitungsvorrichtung (200) mit einer Verzögerung eines Zyklus eines vertikalen Synchronisationssignals der Bildaufnahmeeinheit (130) gesendet wird.

## Revendications

1. Système d'endoscope, comprenant :
une unité d'endoscope (100) ayant une unité de capture d'images (130) pour observer un sujet ;
une mémoire non volatile (150), disposée dans l'unité d'endoscope (100), pour stocker des paramètres spécifiques (500) de l'unité d'endoscope (100) ;
un dispositif de traitement d'images (200) dans lequel au moins l'une d'unités d'endoscope est configurée pour être librement attachable/détachable et qui peut traiter un signal vidéo obtenu par l'unité de capture d'images (130) de l'unité d'endoscope connectée ;
une première unité de transmission de données (231), disposée dans le dispositif de traitement d'images (200), pour transmettre, à l'unité d'endoscope (100), un premier paquet comprenant un signal pour demander les paramètres spécifiques (500) ou des informations en temps réel indiquant un état de fonctionnement de l'unité d'endoscope (100) ;
une première unité de réception de données (232), disposée dans le dispositif de traitement d'images (200), pour recevoir un second paquet qui est transmis par l'unité d'endoscope (100) sur la base du premier paquet et comprend les paramètres spécifiques (500) ou les informations en temps réel ;
une seconde unité de réception de données (121), disposée dans l'unité d'endoscope (100), pour recevoir le premier paquet en provenance du dispositif de traitement d'images (200) ; et
une seconde unité de transmission de données (122) pour transmettre le second paquet dans le format de données sélectionné par l'unité de sélection de données à la première unité de réception de données (232), dans lequel
le premier paquet comprenant des informations d'identification pour demander le second paquet dans le premier format de données comprend des informations de spécification de norme d'images en mouvement pour spécifier une norme vidéo de l'unité de capture d'images (130), et
le premier paquet comprenant des informations d'identification pour demander le second paquet dans le second format de données comprend des informations de commande d'exécution de l'unité d'endoscope (100),
**caractérisé par le fait que**
l'unité d'endoscope (100) comprend en outre une unité de sélection de données (124) pour sélectionner le point de savoir si le second paquet est transféré soit dans un premier format de données pour transférer les paramètres spécifiques (500) au dispositif de traitement d'images (200), soit dans un second format de données pour transférer les informations en temps réel au dispositif de traitement d'images (200) sur la base du signal qui est inclus dans le premier paquet reçu par la seconde unité de réception de données (12) ; et
le dispositif de traitement d'images (200) comprend en outre
une unité de stockage de paramètres (233) pour stocker les paramètres spécifiques (500) transférés en provenance de la mémoire non volatile (150) de l'unité d'endoscope (100) par le second paquet dans le premier format de données, et
une unité de détermination de mise à jour de paramètres (240) pour réaliser un changement partiel des paramètres spécifiques (500) dans l'unité de stockage de paramètres (233) seulement dans un cas dans lequel les informations de résultat d'accès sont correctes lorsque le changement partiel des paramètres spécifiques (500) se produit dans l'unité de stockage de paramètres (233), dans lequel
des informations de requête d'accès partiel pour les paramètres spécifiques (500) de la mémoire non volatile (150) sont réglées en plus des informations de commande d'exécution dans le premier paquet comprenant les informations d'identification pour demander le second paquet dans le second format de données, et
des informations de résultat d'accès pour répondre aux informations de requête d'accès partiel sont réglées dans le second paquet dans le second format de données.

2. Système d'endoscope selon l'une quelconque de la revendication 1, dans lequel
une transmission/réception du premier paquet et du second paquet entre le dispositif de traitement d'images (200) et l'unité d'endoscope (100) est démarrée à la temporisation d'un signal de synchronisation verticale de l'unité de capture d'images (130), et
un transfert du premier paquet et du second paquet est réalisé dans une communication synchrone marche-arrêt en référence à une horloge de fonctionnement de l'unité de capture d'images (130).

3. Système d'endoscope selon la revendication 1, dans lequel le second paquet dans le second format de données dans lequel les informations de résultat d'accès pour répondre aux informations de requête d'accès partiel sont réglées est transmis par l'unité d'endoscope (100) au dispositif de traitement d'images (200) avec un retard à un cycle d'un signal de synchronisation verticale de l'unité de capture d'images (130).

4. Procédé de fonctionnement d'un système d'endoscope ayant une unité d'endoscope (100) comprenant une unité de capture d'mages (130) pour observer un sujet, une mémoire non volatile (150), disposée dans l'unité d'endoscope (100), pour stocker des paramètres spécifiques (500) de l'unité d'endoscope (100), et un dispositif de traitement d'images (200) dans lequel au moins l'une d'unités d'endoscope est configurée pour être librement attachable/détachable et qui peut traiter un signal vidéo obtenu par l'unité de capture d'images (130) de l'unité d'endoscope connectée, le procédé comprenant :
une première étape de transmission de données consistant à transmettre, par le dispositif de traitement d'images (200), à l'unité d'endoscope (100) un premier paquet comprenant un signal pour demander les paramètres spécifiques (500) ou des informations en temps réel indiquant un état de fonctionnement de l'unité d'endoscope (100) ;
une étape de réception de données consistant à recevoir, par l'unité d'endoscope (100), le premier paquet en provenance du dispositif de traitement d'images (200) ;
une étape de transmission de données consistant à transmettre, par l'unité d'endoscope (100), le second paquet sélectionné par l'étape de sélection de données au dispositif de traitement d'images (200) ; et
une étape de réception de données consistant à recevoir, par le dispositif de traitement d'images (200), le second paquet qui est transmis par l'unité d'endoscope (100) sur la base du premier paquet, dans lequel
le premier paquet comprenant des informations d'identification pour demander le second paquet dans le premier format de données comprend des informations de spécification de norme d'images en mouvement pour spécifier une norme vidéo de l'unité de capture d'images (130), et
le premier paquet comprenant des informations d'identification pour demander le second paquet dans le second format de données comprend des informations de commande d'exécution de l'unité d'endoscope (100),
**caractérisé par**
une étape de sélection de données consistant à sélectionner, par l'unité d'endoscope (100), le point de savoir si le second paquet est transféré soit dans un premier format de données pour transférer les paramètres spécifiques (500) au dispositif de traitement d'images (200) lorsque les paramètres spécifiques (500) sont transmis, soit dans un second format de données pour transférer les informations en temps réel au dispositif de traitement d'images lorsque les informations en temps réel sont transmises sur la base du signal qui est inclus dans le premier paquet reçu par l'étape de réception de données dans l'unité d'endoscope (100), dans lequel
des informations de requête d'accès partiel pour les paramètres spécifiques (500) de la mémoire non volatile (150) sont réglées en plus des informations de commande d'exécution dans le premier paquet comprenant les informations d'identification pour demander le second paquet dans le second format de données,
des informations de résultat d'accès pour répondre aux informations de requête d'accès partiel sont réglées dans le second paquet dans le second format de données, et
lorsqu'un changement partiel des paramètres spécifiques (500) se produit dans une unité de stockage de paramètres, disposée dans le dispositif de traitement d'images (200), pour stocker les paramètres spécifiques (500) transférés en provenance de la mémoire non volatile (150) de l'unité d'endoscope (100) par le second paquet dans le premier format de données, le changement partiel des paramètres spécifiques (500) est réalisé dans l'unité de stockage de paramètres seulement dans un cas dans lequel les informations de résultat d'accès sont correctes.

5. Procédé de fonctionnement du système d'endoscope selon la revendication 4, dans lequel
une transmission/réception du premier paquet et du second paquet entre le dispositif de traitement d'images (200) et l'unité d'endoscope (100) est démarrée à la temporisation d'un signal de synchronisation verticale de l'unité de capture d'images (130), et
un transfert du premier paquet et du second paquet est réalisé dans une communication synchrone marche-arrêt en référence à une horloge de fonctionnement de l'unité de capture d'images (130).

6. Procédé de fonctionnement du système d'endoscope selon la revendication 4, dans lequel
le second paquet dans le second format de données dans lequel les informations de résultat d'accès pour répondre aux informations de requête d'accès partiel sont réglées est transmis par l'unité d'endoscope (100) au dispositif de traitement d'images (200) avec un retard à un cycle d'un signal de synchronisation verticale de l'unité de capture d'images (130).
